# EUROPEAN PATENT APPLICATION

(11) **EP 4 451 198 A1**
(43) Date of publication of application: **23.10.2024**
(21) Application number: 22907875.3
(22) Date of filing: 12.12.2022
(51) Int. Cl.: G06Q 50/10, G06Q 10/06, G06Q 10/10, G06Q 50/30, G16H 20/30, G16H 20/70

(54) **METHOD AND APPARATUS FOR PERFORMANCE IMPROVEMENT**

(30) Priority: 15.12.2021 KR 20210180156
(71) Applicant: S-Alpha Therapeutics, Inc., Seoul 06628 (KR)
(72) Inventor: CHOI, Seung Eun, Seoul 06628 (KR); KIM, Myoung Joon, Seoul 06628 (KR); HAHM, Ja Rang, Seoul 06628 (KR); KIM, Tae Hyun, Seoul 06628 (KR)
(74) Representative: Beck Greener LLP
(86) International application number: PCT/KR2022/020158
(87) International publication number: WO 2023/113408

(57) **Abstract**

A method and an apparatus for performance improvement of a user are described in the present application. For example, a digital apparatus for performance improvement of a user comprises: at least one processor; and at least one memory in which an application for the performance improvement of the user is stored, wherein the at least one processor executes the application so as to receive user information from a first user, generate a recommended program for performance improvement of the first user on the basis of the user information, provide the recommended program to the first user, and store a training program for the first user on the basis of a response of the first user.

## Description

### [Technical Field]

This application claims under priority to and benefit of Korean Patent Application No. 10-2021-0180156, filed December 15, 2021, the disclosure of which are incorporated herein by reference in their entireties.

### [Background Art]

Anxiety disorder is a type of mental disorder of which the feeling of anxiousness or fear for something that has not happened yet has been pathologically strong or long-lasting to disrupt one's daily life. A stress is a type of reaction that occurs to protect one's body when threatened or challenged by an outside factor, i.e., a stressor. It is difficult to eliminate the root cause of anxiety or stress itself in physiologically. Moreover, constant and excessive stress status may also disrupt one's daily life, developing into 'Neurotic, stress-related and somatoform disorders. Accordingly, the feelings of anxiety or stress may significantly affect one's performance or ability to do something important in their life. For example, the feeling of anxiety decreases an athlete's performance ability, leading to unsatisfied results without showing the athlete's performance to the fullest.

Existing anxiety or stress coping methods are mostly involved in meditation, psychological relaxation, psychotherapy, game, mindfulness, stress management, etc. These methods are neither developed considering the user's neurohumoral factors and neuroplasticity, nor based on objective, scientific theory and evidence to cope with the anxiety and stress. The users rely heavily on subjective reviews of other reviewers rather than evidence-based evaluation. Thus, methods and apparatuses that cope with anxiety and/or stress based on neurohumoral factors and neuroplasticity thereby enabling evidence-based evaluation are needed.

### [Disclosure]

### [Technical Solution]

Methods and apparatuses are described herein for enhancing performance. For example, an apparatus may generate one or more performance enhancing instructions that are designed to cause, based on the user's performance of the one or more performance enhancing instructions, at least one physiological changes in the user's physiological mechanism to cope with at least one of anxiety or stress. The apparatus may then provide the user the one or more performance enhancing instructions via the apparatus. The at least one physiological changes may include at least one of dopamine secretion induction, oxytocin secretion induction, cortisol level reduction, melatonin secretion induction, or synapsing promotion. The one or more performance enhancing instructions comprise a first concentration enhancing digital instruction related to the dopamine secretion induction, a second concentration enhancing digital instruction related to the melatonin secretion induction, an anxiety/stress reducing digital instruction related to at least one of the oxytocin secretion induction or the cortisol level reduction, and a coordination & agility enhancing digital instruction related to the synapsing promotion.

### [Description of Drawings]

A more detailed understanding may be had from the following description, given by way of example in conjunction with the accompanying drawings, wherein like reference numerals in the figures indicate like elements, and wherein:
FIG. 1 is a diagram illustrating an example mechanism of action (MOA) for enhancing performance;
FIG. 2 is a diagram illustrating an example anticipated neurohumoral status change after inducing positive neurotranmitters and/or synapsing;
FIG. 3 is a diagram illustrating an example maximization of the performance of an athlete;
FIG. 4 is a diagram illustrating an example physiological mechanism decreasing an athlete's performance due to anxiety/stress;
FIG. 5 is a diagram illustrating an example MOA of overcoming an athlete's anxiety through instructions;
FIG. 6 is a diagram illustrating an example patient assistance in the recovery from the operation of intraocular lens (IOL) implants/ multifocal lens in ophthalmology;
FIG. 7 is a diagram illustrating an example physiological mechanism of forming anxiety that can develop in a patient's body undergoing ophthalmology operation before and/or after the surgery;
FIG. 8 is a diagram illustrating an example MOA of overcoming a patient's anxiety through behavioral language;
FIG. 9 is a diagram illustrating an example performance enhancing application (PEA) that copes with anxiety and stress of users;
FIG. 10 is a diagram illustrating an example of performance enhancing application (PEA);
FIG. 11 is a diagram illustrating examples of instruction input and practice monitoring of performance enhancing application (PEA); and
FIG. 12 is a system diagram illustrating an example device that can be used for performance enhancing application (PEA).
FIG. 13 is a diagram illustrating stressor disease diagnostic criteria for performance enhancing application (PEA) and relationship with sensors.
FIG. 14 is a diagram illustrating a data sensing standardization for performance enhancing application (PEA).
FIG. 15 is a diagram illustrating physical part analysis using motion detection.
FIG. 16 is a diagram illustrating a process of setting goals for athletes using performance enhancing application (PEA).
FIG. 17 is a block diagram illustrating a system of a performance enhancement application (PEA).
FIG. 18 is a diagram illustrating a timeline of a performance enhancement application (PEA).
FIG. 19 is a diagram illustrating a sport setup screen of a performance enhancement application (PEA).
FIG. 20 is a diagram illustrating a goal setting screen of a performance enhancement application (PEA).
FIG. 21 is a diagram illustrating a main screen of a performance enhancement application (PEA).
FIG. 22 is a diagram illustrating example screens of a game preparation program.
FIG. 23 is a diagram illustrating an example screen when a user performs a game preparation program.
FIG. 24 is a diagram illustrating example screens of a goal management program.
FIG. 25 is a diagram illustrating an example screen when a user performs a goal management program.
FIG. 26 is a diagram illustrating example screens of a mental coaching program.
FIG. 27 is a diagram illustrating example screens of image training in a mental coaching program.
FIG. 28 is a diagram illustrating example screens for creating a new image training screen in a mental coaching program.
FIG. 29 is a diagram illustrating example screens for a user to add a new training program.
FIG. 30 is a diagram illustrating example screens for a user to create a new training program.
FIG. 31 is a diagram illustrating example screens for requesting permission from a coach when a user creates a new training program.
FIG. 32 is a diagram illustrating example screens for a user to add a game or program on the schedule screen.
FIG. 33 is a diagram illustrating example screens for a user to add a game on the schedule screen.
FIG. 34 is a diagram illustrating example screens for a user to view a diary for a particular day on the schedule screen.
FIGS. 35 and 36 are diagrams illustrating example screens for a user to communicate with an administrator through a performance enhancement application (PEA).
FIG. 37 is a diagram illustrating example screens for a user to view program usage statistics.
FIG. 38 is a diagram illustrating example screens for a user to view notifications within a performance enhancement application (PEA).
FIGS. 39 to 41 are diagrams illustrating example processes of a performance enhancement application (PEA) that may be performed by a processor.

### [Mode for Invention]

Although the terms first, second, etc. may be used to describe various elements, these elements are not limited by these terms. These terms are only used to distinguish one element from another. For example, a first element could be termed a second element, and, similarly, a second element could be termed a first element, without departing from the scope of exemplary embodiments. The term "and/or" includes any and all combinations of one or more of the associated listed items.

The terminology used herein is for the purpose of describing particular embodiments only and is not intended to be limiting of exemplary embodiments. The singular forms "a," "an" and "the" are intended to include the plural forms as well, unless the context clearly indicates otherwise. It will be further understood that the terms "comprises," "comprising," "includes" and/or "including," when used herein, specify the presence of stated features, integers, steps, operations, elements, components and/or groups thereof, but do not preclude the presence or addition of one or more other features, integers, steps, operations, elements, components and/or groups thereof.

Diagnostic and Statistical Manual of Mental Disorders (DSM), published by the American Psychiatric Association, is most widely used in terms diagnosing mental disorders. According to the classification of DSM-V (5^{th} edition), anxiety disorder is a type of mental disorder of which the feeling of anxiousness or fear for something that has not happened yet has been pathologically strong or long-lasting to disrupt one's daily life. It includes generalized anxiety disorder, a phobia (specific phobia, social phobia, agoraphobia), panic disorder, obsessive-compulsive disorder, post-traumatic stress disorder, and separation anxiety disorder. If left untreated, it can lead to depression and addiction, and can be a risk factor of dementia or suicide.

Even if not pathologically diagnosed as an anxiety disorder, an average person may feel particularly anxious in certain situations, and the uncontrolled feeling of anxiousness sometimes lasts in undesirable actions/results.

Currently, a number of anxiety (allegedly) reducing applications are available on Google App Store, most of which are developed involving meditation, psychological relaxation, psychotherapy, brain exercise, meta cognition, mind control, etc. These apps target unspecified individuals, and the users rely heavily on subjective reviews of other reviewers rather than evidence-based evaluation to use the apps.

A stress reaction is a type of reaction that occurs to protect one's body when threatened or challenged by an outside factor, i.e., a stressor. In a well-managed stress status, the feeling of tension causes stress hormones to awaken the brain and thereby to help improve memory. Also, in the incipient stages, cortisol, a type of stress hormone, enhances the body's immunity. However, the 10th revision of the International Statistical Classification of Diseases and Related Health Problems (ICD-10) has classified constant and excessive stress status into 'Neurotic, stress-related and somatoform disorders (F40-F48)'.

Aside from these pathological stress statuses, for instance, an athlete may fail to show better performance in an important game due to excessive mental stress.

Currently, numerous anti-stress or stress (allegedly) reducing applications are available on Google App Store, most of which are developed involving meditation, relaxation, funny game, psychotherapy, mindfulness, stress management, etc. These apps target unspecified individuals, and the users rely heavily on subjective reviews of other reviewers rather than evidence-based evaluation to use the apps.

Neuroplasticity refers to the human brain's ability to change throughout one's life experiences. Results of recent researches demonstrate that learning or other environmental factors make neurons continuously develop or deteriorate. Particularly, the hippocampus, a part of the brain responsible for memory, enhances neuroplasticity significantly by incessantly developing new neurons and deteriorating the old neurons. The frontal area of brain is the location of active neuronal myelination, resulting in synapsing and neuroplasticity too. The BDNF protein is known to target the synapses of neurons especially to promote neuroplasticity.

In particular, myelin formation and remodeling of oligodendrocyte, which has been developed from oligodendrocyte precursor cells (OPC), plays a very important role in terms of learning, exercising, and making up for the impaired brain regions. Increasing neuroplasticity leads to neurons' synapsing and thereby contributes to the enhancement of coordination and agility.

At the status quo, there is no application which applies the notion of neuroplasticity with the enhancement of coordination and agility. There are only applied cases where brain training and brain game are used at a rudimentary level.

In order to overcome these limitations of the existing apps, 1) target users of the app need to be specified, 2) evidence-based evaluation needs to proceed through app development based on the hypothesis which has been through medical evidences and scientific ones.

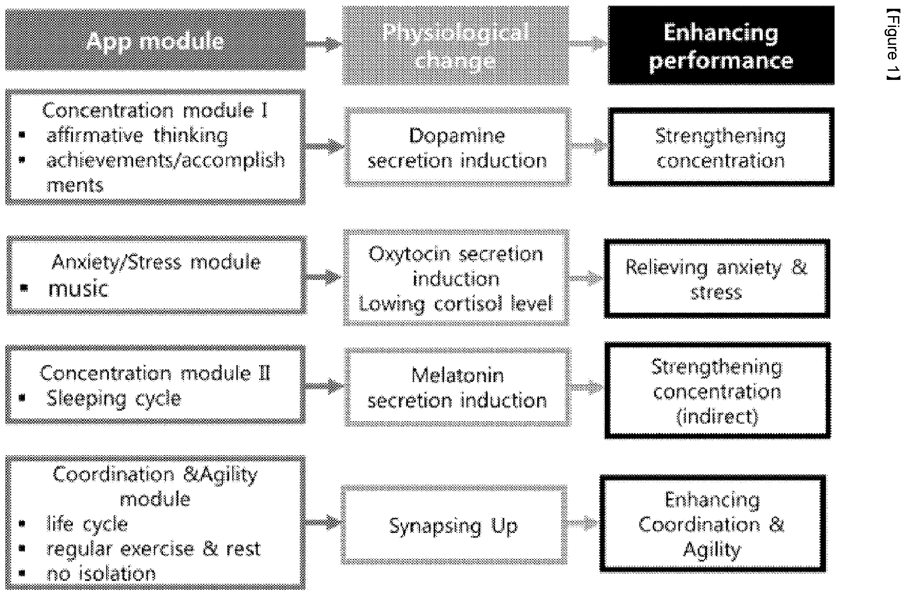

FIG. 1 illustrates an example mechanism of action (MOA) for enhancing performance, which may be used in combination with any of other embodiments described herein.

By practicing the instructions that have been delivered to the user via the application, neurohumoral factors and neuroplasticity, which cope with anxiety and stress in physiological terms, increase. This, in turn, improves the user's concentration, overcome anxiety and pressure, and enhances coordination and agility. Consequently, the user's performance can be enhanced.

It is difficult to eliminate the root cause of anxiety or stress itself in physiologically, except for pathological anxiety disorder and neurological stress. For example, although an athlete may undergo significant anxiety and stress before athletics or game, patient before a scheduled operation, an artist before a recital/show, and a student before an exam, feeling anxiety and stress is a part of the athletics/recovery/recital/show/exam/etc. in terms of physiological process.

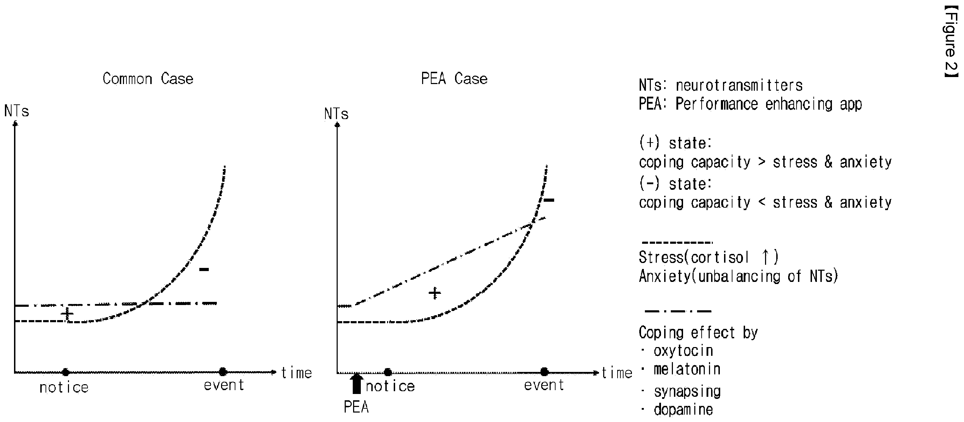

FIG. 2 illustrates an example anticipated neurohumoral status change after inducing positive neurotransmitters and/or synapsing, which may be used in combination with any of other embodiments described herein. As illustrated in FIG. 2, even under the condition where there are limits in eliminating the root cause of anxiety or stress, the negative impacts of anxiety or stress may be reduced by "coping with" or boosting up positive neurotransmitters (e.g., dopamine, oxytocin, and/or melatonin) or synapsing.

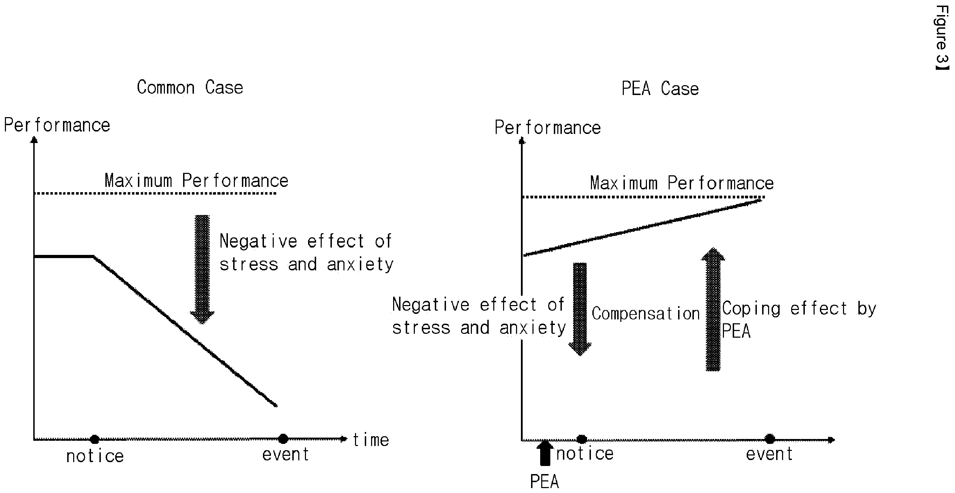

FIG. 3 illustrates an example maximization of the performance of an athlete, which may be used in combination with any of other embodiments described herein. Athletes may undergo significant anxiety prior to athletics or major games. Anxiety may be triggered as a result of the combination of possibilities where he might lose the tournament in athletics or make mistakes, concerns of potential criticism from audiences, and a sense of duty to win the game. The feeling of anxiety decreases the athlete's performance ability, leading to unsatisfiable results without showing the athlete's performance to the fullest.

By using the performance enhancing app (PEA), athletes may cope with anxiety and stress to enhance concentration, coordination, and agility, and reach his/her fullest potential.

The existing methods to overcome athletes' anxiety consists of relaxation training, thought stopping, labeling, and deep breathing along with sufficient sleeping, exercise, complex carbohydrates intake, caffeine/sugar intake limit, etc. The relaxation training includes yoga, meditation, breathing exercise, pilates, etc. Thought stopping includes changing negative thoughts into positive thoughts and task-centered approach rather than result-centered. Labeling includes letting one perceive his/her own thoughts and emotions.

However, the above method, for example, to overcome sports performance anxiety, do not compensate for the changes in physiological neurohumoral factors of the body occurring before the athlete's big/important games. In other words, it is symptomatic therapy for anxiety.

Meanwhile, the physiological mechanism for an athlete to overcome anxiety such as sufficient sleeping, exercise, complex carbohydrates intake, caffeine/sugar intake limit and others has not yet been specifically identified.

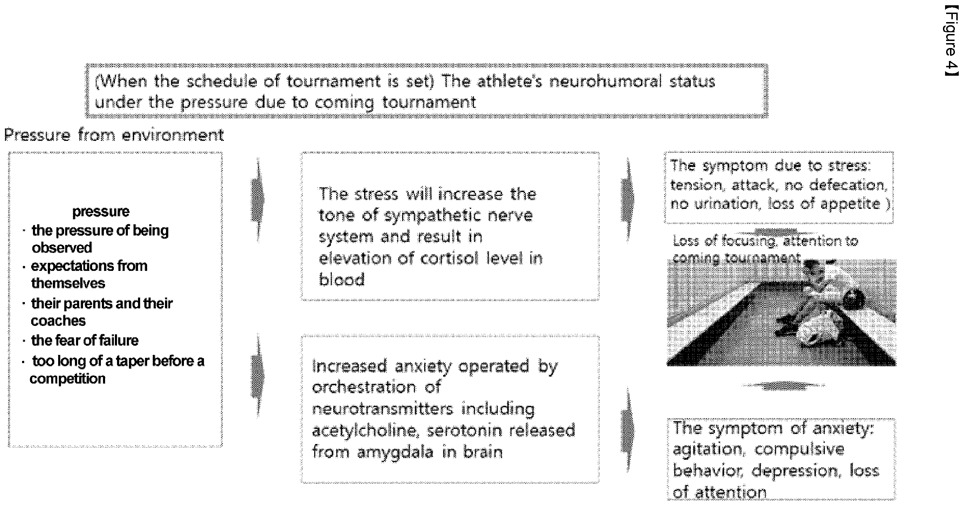

FIG. 4 illustrates an example physiological mechanism decreasing an athlete's performance due to anxiety/stress before games, which may be used in combination with any of other embodiments described herein. As illustrated in FIG. 4, the athletes' anxiety may be formed before games/athletics. This kind of anxiety formation may involve orchestration of neurotransmitters (NTs), and hence it may be difficult to identify a single, particular NT as the major source of the anxiety formation.

Among various NTs which cause anxiety, four NTs may be suggested as the neurohumoral factors which can result in coping over anxiety formation, i.e., increasing of dopamine, increasing of oxytocin, decreasing of cortisol, increasing of melatonin. In addition, synapsing regarding brain myelin formation and remodeling may also be suggested as the neuronal, cellular, and molecular mechanism of inducing coping-skill over anxiety formation.

The control using drugs of the present mechanism is not likely to be accounted as antagonizing mechanism, due to the fact that the relevant NTs with anxiety/stress formation are the most basic NTs to maintain the body's homeostasis in physiological terms. In other words, it may bring serious side-effect and safety issues, provided that it could be a target molecule to overcome anxiety. Even pushing aside these difficulties, the control of NT using drugs cannot be a solution to athletes' anxiety because it may trigger doping issues.

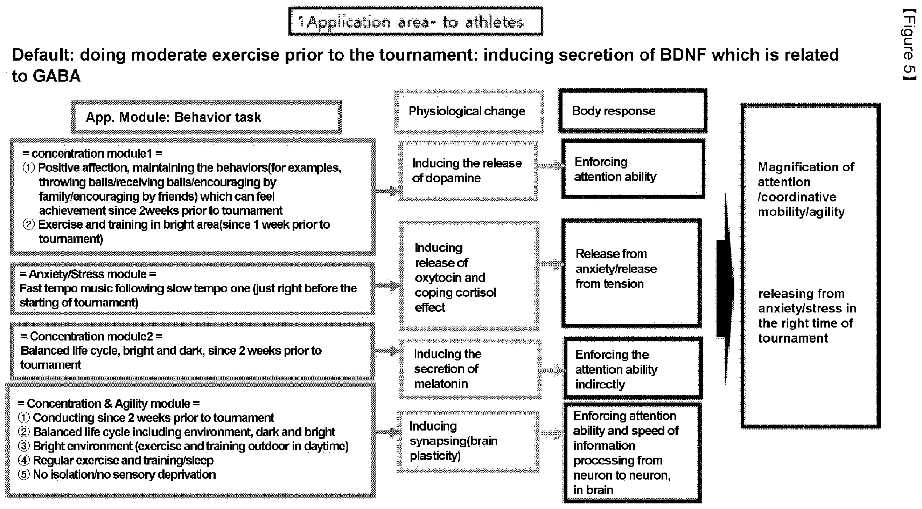

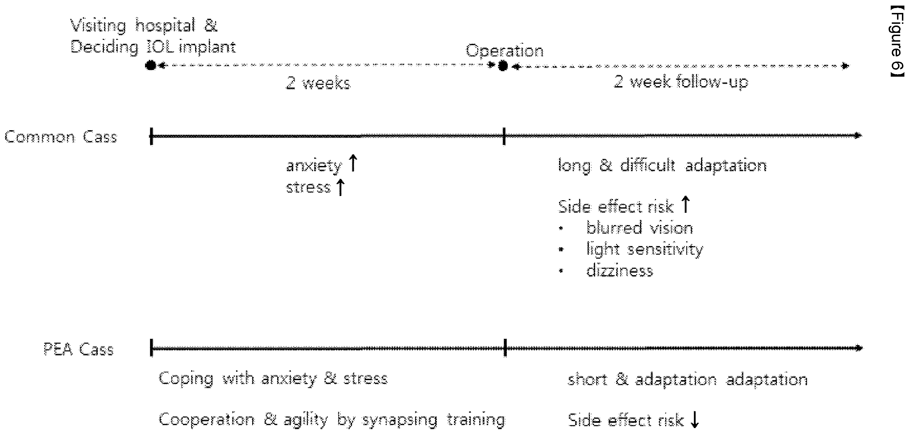

FIG. 5 illustrates an example MOA of overcoming an athlete's anxiety through instructions, which may be used in combination with any of other embodiments described herein. For example, an athlete's performance and ability may be maximized through instruction-practice which is delivered to the athlete via the application for two weeks prior to the game, based on the physiological mechanism of athletes' anxiety and stress formation. The MOA (mode of action or mechanism of action) to overcoming athletes' anxiety through the behavioral language may be illustrated as in FIG. 5. The MOA may comprise one or more modules (e.g., four modules). Specifically, concentration module 1 may include, but are not limited to: positive thinking, and executing instructions (e.g., for 2 weeks) that can make one feel a small sense of achievement constantly (e.g., throwing balls, cheer from family members, or cheer from friends.). Anxiety/Stress module may include, but are not limited to: listening up-tempo music after down-tempo music (e.g., before the start of the game). Concentration module 2 may include, but are not limited to, providing normal day and night environment (e.g., for 2 weeks). Coordination & Agility module may include, but are not limited to: starting the module at least 2 weeks earlier to the game, having proper life cycle, daylight (e.g., outdoor exercise during the day), regular exercise and sleeping, and no isolation/no sensory deprivation. The corresponding changes in NT and synapsing are summarized in FIG. 6 physiological changes column (the green box). These physiological changes are changed into physical changes (the black box in Fig. 6), and eventually, an athlete can relieve anxiety and pressure during the game and maximize his/her concentration, coordination, and agility.

In one aspect, the present disclosure relates to a method of enhancing performance of a user, the method comprising providing, by an electronic device to the user, one or more first modules selected from the group consisting of a concentration module I for inducing dopamine secretion, an anxiety and/or stress module, a concentration module II for inducing melatonin secretion, and a coordination and agility module, each of the one or more first modules comprising one or more first instructions for the user to follow. In some embodiments, the electronic device (i) comprises a sensor sensing adherence by the user to the first instructions of the one or more first modules, (ii) transmits adherence information based on the adherence, to a server, and (iii) receives one or more second instructions from the server based on the adherence information. In additional embodiments, the method further comprise providing, by the electronic device to the user, one or more second modules selected from the group consisting of a concentration module I for inducing dopamine secretion, an anxiety and/or stress module, a concentration module II for inducing melatonin secretion, and a coordination and agility module, the one or more second modules comprising the one or more second instructions.

In some embodiments, said one or more first modules comprise at least two, three or four of a concentration module I for inducing dopamine secretion, an anxiety and/or stress module, a concentration module II for inducing melatonin secretion, and a coordination and agility module. In some embodiments, said one or more first modules comprise all of a concentration module I for inducing dopamine secretion, an anxiety and/or stress module, a concentration module II for inducing melatonin secretion, and a coordination and agility module. In some embodiments, said one or more second modules comprise at least two, three or four of a concentration module I for inducing dopamine secretion, an anxiety and/or stress module, a concentration module II for inducing melatonin secretion, and a coordination and agility module. In some embodiments, said one or more second modules comprise all of a concentration module I for inducing dopamine secretion, an anxiety and/or stress module, a concentration module II for inducing melatonin secretion, and a coordination and agility module.

In some embodiments, the one or more first modules comprise the concentration module I to strengthen concentration of the user. In some embodiments, the concentration module I comprises one or more first instructions directed to affirmative thinking, achievement, and accomplishment. In additional embodiments, the one or more instructions comprise one or more instructions for positive affection, maintaining behaviors, exercise, and training in a bright area. In further embodiments, the concentration module I includes instructions to train in a bright area at least one week before a performance.

In some embodiments, the one or more first modules comprise the anxiety and/or stress module to relieve anxiety and stress. In some embodiments, the anxiety and/or stress module comprises one or more first instructions to induce oxytocin secretion and/or lower cortisol level. In additional embodiments, the one or more first instructions comprise one or more instructions to listen to music. In further embodiments, the electronic device receives and plays the sounds of the music. In yet further embodiments, the one or more first instructions comprise one or more instructions to listen to music in less than two or one hours before the performance.

In some embodiments, the one or more first modules comprise the concentration module II to strengthen concentration of the user. In some embodiments, and the concentration module II comprises one or more first instructions directed to sleeping cycle. In additional embodiments, the one or more instructions comprise one or more instructions for a balanced life cycle and brightness of the user's environment. In further embodiments, the one or more instructions comprise one or more instructions for sleeping cycle at least one or two weeks before the performance.

In some embodiments, the one or more first modules comprise the coordination and agility module to enhance coordination and agility. In some embodiments, and the coordination and agility module comprises one or more first instructions to promote synapsing. In additional embodiments, the one or more first instructions comprise one or more instructions for a balanced life cycle, a regular exercise and rest, brightness of the user's environment, and no isolation or sensory deprivation. In further embodiments, the coordination and agility module is provided to the user at least one or two weeks before a performance.

In some embodiments, the adherence is an adherence to the first instructions of the concentration module I, and the adherence information based on the adherence to the first instructions of the concentration module I is used to generate the second instructions only of the concentration module I. In some embodiments, the adherence is an adherence to the first instructions of the anxiety and/or stress module, and the adherence information based on the adherence to the first instructions of the anxiety and/or stress module is used to generate the second instructions only of the anxiety and/or stress module. In some embodiments, the adherence is an adherence to the first instructions of the concentration module II, and the adherence information based on the adherence to the first instructions of the concentration module II is used to generate the second instructions only of the concentration module II. In some embodiments, the adherence is an adherence to the first instructions of the a coordination and agility module, and the adherence information based on the adherence to the first instructions of the coordination and agility module is used to generate the second instructions only of the a coordination and agility module.

In some embodiments, the user is a musician, and the performance is musical performance. In some embodiments, the user is an athlete, and the performance is athletic performance. In some embodiments, the user is a baseball player. In some embodiments, the user is a skier.

In some embodiments, the user is a patient who underwent the operation of Intraocular lens/multifocal lens implant in ophthalmology. Embodiments for a patient who underwent the operation of Intraocular lens/multifocal lens implant in ophthalmology are described herein.

FIG. 6 illustrates an example patient assistance in the recovery from the operation of intraocular lens (IOL) implants/ multifocal lens in ophthalmology, which may be used in combination with any of other embodiments described herein.

IOL implants surgery usually takes about 2 weeks of waiting period after getting admission approval notice for surgery in ophthalmology area, until the actual surgery takes place. During this period, some patients feel extremely anxious, which negatively influences on postop recovery and satisfaction of operation result. To add on, there is a need for auxiliary measures to help patients overcome the side-effects after the surgery and adjust to IOL.

The performance enhancing application (PEA) described herein has positive impacts on enhancing postop recovery from blurry vision and satisfaction about surgery itself to maximizing neuroplasticity, inducing release of BDNF as well as to relieve patients' anxiety over the surgery through coping skills, by doing instructions practiced for 2 weeks before or after the surgery.

In the present disclosure, athletes and IOL implants inserted into patients are exemplified to describe the details of the invention, however, the present invention is not limited to the cases of athletes and IOL implants inserted patients. The target users may include, but are not limited to, artists, students, singers, contestees, and others who have to show performances after a given period of time.

For IOL implants surgery, an education program on wearing an eye patch for protecting the affected part after the surgery, applying eyewash, eye exercises, eye health and others has been conducted before the surgery depending on each hospital's condition. However, as the education program takes place right before the surgery, it is reported to be not as effective, worsening the patients' anxiety.

Therefore, there is a need for education programs after the surgery at an appropriate time which can reduce patients' anxiety for the surgery and at the same time helps the effective management of the patients. However, such endeavors to reduce anxiety before and after the eye surgery have merely been made so far.

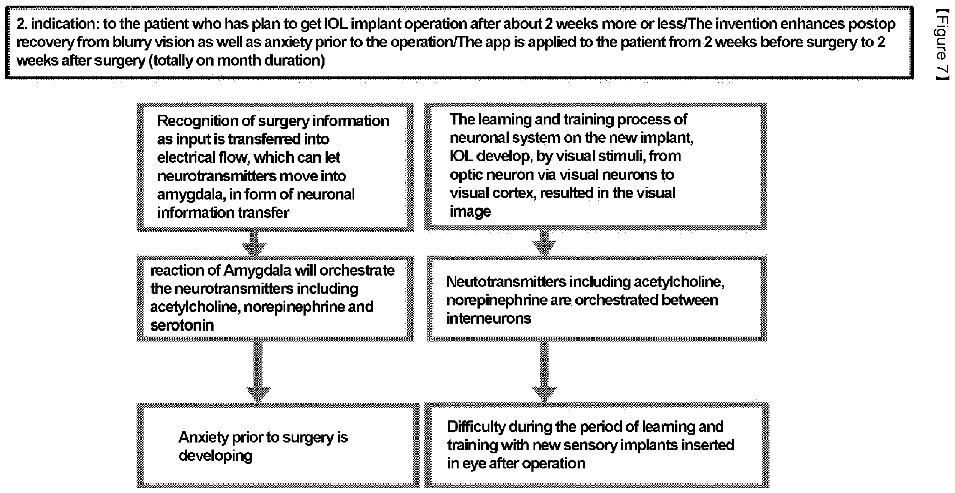

FIG. 7 illustrates an example physiological mechanism of forming anxiety that can develop in a patient's body undergoing ophthalmology operation before and/or after the surgery, which may be used in combination with any of other embodiments described herein. As illustrated in FIG. 7, patient's anxiety may be formed before, during and/or after the surgery. The formation of anxiety may be affected by the orchestration of various NTs, and thus it is difficult to define a single, particular NT as the mechanism of the anxiety formation.

The physiological mechanism of resulting in coping with an athlete's anxiety formation and that of anxiety formed by a patient who will undergo ophthalmology surgery with local anesthesia are the same or substantially similar. The four NTs - increasing of dopamine secretion, increasing of oxytocin secretion, decreasing or suppression of the effect of cortisol, increasing of melatonin secretion - used in physiological mechanism which can induce coping skill over athletes' anxiety/stress formation may be suggested as the neurohumoral factors of coping over anxiety/stress formation. In addition, the synapsing related to brain myelin formation and remodeling may be suggested as the cellular and molecular mechanism which can induce coping skill over anxiety/stress formation.

The relevance of each factor which can induce coping skill over anxiety/stress formation is described above as in the physiological mechanism which can induce coping skill over athletes' anxiety/stress formation.

The control of the physiological mechanism can induce coping skill over anxiety/stress formation through behavioral language. The control using drugs of the present mechanism is not likely to be accounted as antagonistic due to the fact that the relevant NTs are the most basic NTs to maintain the body's homeostasis in physiological terms. Anxiety/stress coping involving the application, unlike drug prescriptions, can prevent side-effect and safety issues.

In an embodiment, it may take up to four weeks (e.g., (two weeks each, before and after the surgery) for a patient who had IOL surgery to overcome the patients' anxiety through instruction-practice which is delivered to the patient via the application. Once the operation schedule is set for treatment, and pre-operation or post-operation care is needed before or after surgery using the application described herein. The application may be based on the physiological mechanism of patients' anxiety/stress formation.

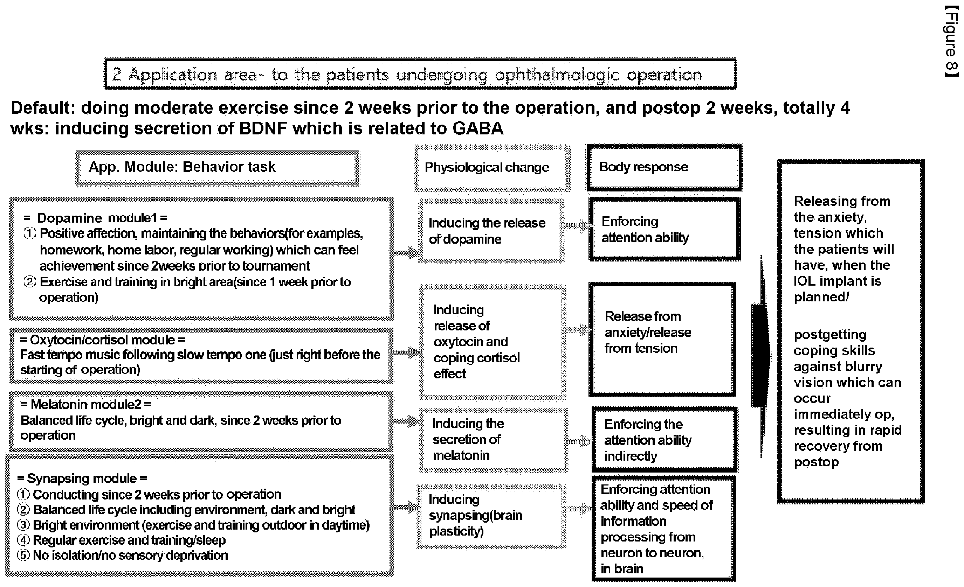

FIG. 8 illustrates an example MOA of overcoming a patient's anxiety through behavioral language, which may be used in combination with any of other embodiments described herein. The MOA may comprise one or more modules (e.g., four). For example, dopamine module may include, but are not limited to, positive thinking, executing instructions (for 2 weeks) that can make one feel a small sense of achievement constantly (doing chores, continuing with the job, etc.), and maintaining the life style in the light (starting from a week before the surgery). Oxytocin/Cortisol module may include, but are not limited to, listening up-tempo music after down-tempo music (e.g., a few hours earlier to the surgery). Melatonin module may include, but are not limited to, providing normal day and night environment (e.g., 2 weeks before the surgery). Synapsing module may include, but are not limited to, starting the module at least 2 weeks before the surgery, having proper life cycle, daylight (e.g., outdoor exercise during the day), regular exercise and sleeping, and no isolation/no sensory deprivation. The corresponding changes in NT and synapsing are summarized in FIG. 8 physiological changes column (the green box). These physiological changes are changed into physical changes (the black box in FIG. 8), and eventually, a patient can relieve anxiety before and after the surgery and minimize the side-effects of the surgery.

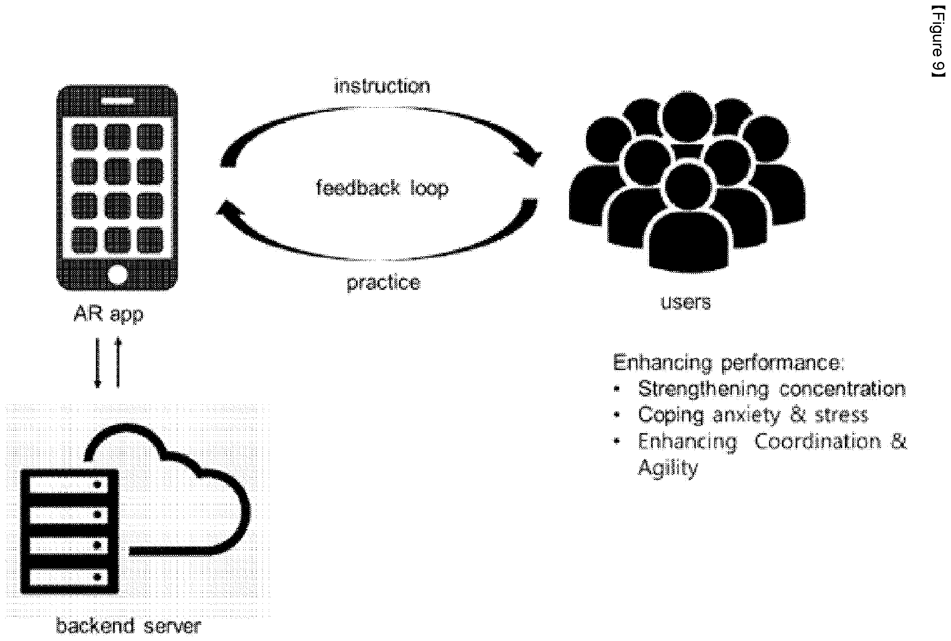

FIG. 9 illustrates an example performance enhancing application (PEA) that copes with anxiety and stress of a user, which may be used in combination with any of other embodiments described herein. The process of which the performance enhancing application (PEA) reduces the users' anxiety is illustrated in FIG. 9. The performance enhancing application (PEA) may be embodied or implemented in the form of application in digital devices such as a desktop computer, a laptop computer, a tablet, a smartphone, an loT device, a wireless transmit/receive unit (WTRU) or the like. A user may download the application, receive instructions via performance enhancing application (PEA) III, and practice them. The user's practice may give feedback to the application through various means, for example, passive data gathering using log-in/out information, user review/evaluation, sensors, etc. The instruction-practice feedback loop may increase the users' adherence to induce their constant and voluntary participation. Taking a closer look at the specific physiological responses by the performance enhancing application (PEA), the following may be induced: secretion of dopamine, oxytocin and melatonin, suppression of cortisol, and neurons' synapsing as a result of the instruction-practice process. The performance enhancing application (PEA) may be designed based on the MOA that induces the aforementioned physiological responses. Consequently, a user such as an athlete or a patient can actively participate in overcoming anxiety or stress through the practiced instructions to maximize performance abilities with enhanced concentration, coordination and agility.

In some embodiments, the server disclosed herein receives the one or more second instructions from an external reviewer. In additional embodiments, the external reviewer comprises a health professional. In additional embodiments, the external reviewer comprises an artificial intelligence (Al).

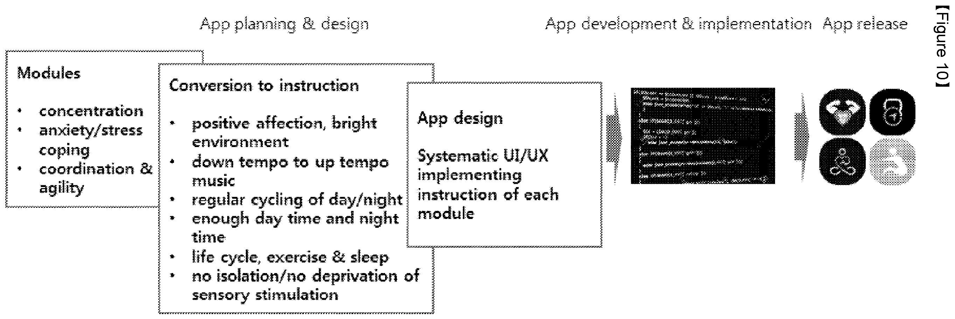

FIG. 10 illustrates an example of performance enhancing application (PEA), which may be used in combination with any of other embodiments described herein. performance enhancing application (PEA) may specify the instructions of the concentration, anxiety/stress coping, coordination & agility modules as described in FIGs. 5 and 8, and reflect them in the application design. The performance enhancing application (PEA) can be made and be launched based on the application programming that reflects the aforementioned application planning.

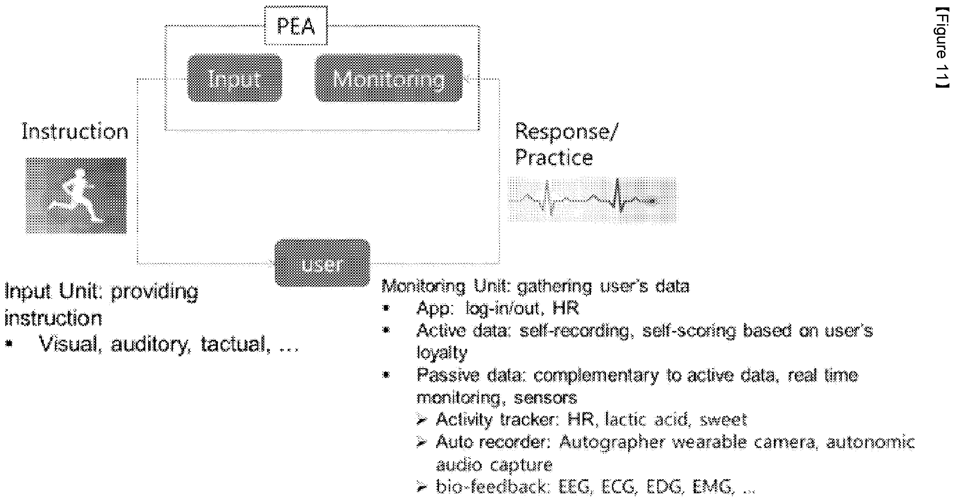

FIG. 11 illustrates examples of instruction input and practice monitoring of performance enhancing application (PEA), which may be used in combination with any of other embodiments described herein. The instruction input may be provided to a user by visual display, auditory narration, touch/vibration, etc. The practice out can be classified to three categories: 1) application log-in/out information, 2) active data which are generated by user's typing or recording, 3) passive data which are gathered by sensors. The sensors for passive data gathering include activity trackers, auto recorders, bio-feedback instruments.

In some embodiments, the sensor described herein comprises one or more of: a motion sensor, a camera, an accelerometer, a magnetometer, a light sensor, a microphone, a proximity sensor, a touch sensor, a gyroscope, a Global Positioning System (GPS) sensor, an ambient light sensor, a fingerprint sensor, a pedometer, a heart rate sensor, and a thermometer. In additional embodiments, the sensor comprises the GPS sensor and the heart rate sensor. In further embodiments, the sensor comprises a touch sensor, and the user provides the adherence information to the electronic device using the touch sensor.

In some embodiments, the sensor described herein detects positions of knees and/or ankles of the user, and/or the distances thereof. In further embodiments, the sensor described herein detects positions of the neck, shoulder, arms and/or hip of the user, and/or the distances thereof. In further embodiments, the sensor described herein detects positions of the fingers of the user, and/or the distances thereof.

In some embodiments, the sensor captures motions of the athletic performance. In additional embodiments, the sensor captures at least 5, 10, 15, 20, 25, or 30 images per second.

In another aspect, the present disclosure is directed to a system for enhancing performance of a user, comprising: a digital apparatus configured to execute a digital application comprising one or more first modules described herein, for enhancing performance of a user, wherein the digital apparatus comprises a sensor described above for sensing adherence by the user to a first set of instructions of the one or more first modules. In some embodiments, the system also comprise a healthcare provider portal configured to provide one or more options to a healthcare provider to perform one or more tasks to prescribe performance enhancement of a user based on information received from the digital application. In some embodiments, the system also comprise an administrative portal configured to provide one or more options to an administrator of the system to perform one or more tasks to manage access to the system by the healthcare provider. In additional embodiments, the digital application instructs a processor of the digital apparatus to execute operations comprising: generating digital therapeutic modules for enhancing performance based on a mechanism of action in and a therapeutic hypothesis for the enhancing performance of a user. In further embodiments, the generating of the digital therapeutic modules comprises generating the digital therapeutic modules based on biochemical factors related to enhance performance of a user.

In some embodiments, the one or more options provided to the healthcare provider are selected from the group consisting of adding or removing the user, viewing or editing personal information for the user, viewing adherence information for the user, viewing a result of the user for one or more at least partially completed digital therapeutic modules, prescribing one or more digital therapeutic modules to the user, altering a prescription for one or more digital therapeutic modules, and communicating with the user.

In some embodiments, the one or more options comprise the viewing or editing personal information for the user, and the personal information comprises one or more selected from the group consisting of an identification number for the user, a name of the user, a date of birth of the user, an email of the user, an email of the guardian of the user, a contact phone number for the user, a prescription for the user, and one or more notes made by the healthcare provider about the user.

In some embodiments, the personal information comprises the prescription for the user, and the prescription for the user comprises one or more selected from the group consisting of a prescription identification number, a prescription type, a start date, a duration, a completion date, a number of scheduled or prescribed digital therapeutic modules to be performed by the user, and a number of scheduled or prescribed digital therapeutic modules to be performed by the user per day.

The system of any one of embodiments 43-70, wherein the one or more options comprise the viewing the adherence information, and the adherence information of the user comprises one or more of a number of scheduled or prescribed digital therapeutic modules completed by the user, and a calendar identifying one or more days on which the user completed, partially completed, or did not complete one or more scheduled or prescribed digital therapeutic modules.

In some embodiments, the one or more options comprise the viewing the result of the user, and the result of the user for one or more at least partially completed digital therapeutic modules comprises one or more selected from the group consisting of a time at which the user started a scheduled or prescribed digital therapeutic module, a time at which the user ended a scheduled or prescribed digital therapeutic module, an indicator of whether the scheduled or prescribed digital therapeutic module was fully or partially completed, and an exercise intensity (EI).

In some embodiments, the one or more options provided to the administrator of the system are selected from the group consisting of adding or removing the healthcare provider, viewing or editing personal information for the healthcare provider, viewing or editing de-identified information of the user, viewing adherence information for the user, viewing a result of the user for one or more at least partially completed digital therapeutic modules, and communicating with the healthcare provider.

In some embodiments, the one or more options comprise the viewing or editing the personal information, and the personal information of the healthcare provider comprises one or more selected from the group consisting of an identification number for the healthcare provider, a name of the healthcare provider, an email of the healthcare provider, and a contact phone number for the healthcare provider.

In some embodiments, the one or more options comprise the viewing or editing the de-identified information of the user, and the de-identified information of the user comprises one or more selected from the group consisting of an identification number for the user, and the healthcare provider for the user.

In some embodiments, the one or more options comprise the viewing the adherence information for the user, and the adherence information of the user comprises one or more of a number of scheduled or prescribed digital therapeutic modules completed by the user, and a calendar identifying one or more days on which the user completed, partially completed, or did not complete one or more scheduled or prescribed digital therapeutic modules.

In some embodiments, the one or more options comprise the viewing the result of the user, and the result of the user for one or more at least partially completed digital therapeutic modules comprises one or more selected from the group consisting of a time at which the user started a scheduled or prescribed digital therapeutic module, a time at which the user ended a scheduled or prescribed digital therapeutic module, an indicator of whether the scheduled or prescribed digital therapeutic module was fully or partially completed, and an exercise intensity (EI).

In some embodiments, the digital application further comprises a push alarm and/or push notifications for reminding the subject to complete a digital therapeutic module.

In some embodiments, the digital apparatus comprises: a digital instruction generation unit configured to generate digital therapeutic modules for enhancing performance of the user, generate digital instructions based on the digital therapeutic modules, and provide the digital instructions to the user; and an outcome collection unit configured to collect the user's execution outcomes of the digital instructions. In additional embodiments, the digital instruction generation unit generates the digital therapeutic modules based on the inputs from the healthcare provider. In additional embodiments, the digital instruction generation unit generates the digital therapeutic modules based on information received from the user.

In some embodiments, the information is received from the user comprises at least one of basal factors, medical information, and digital therapeutics literacy of the user, the basal factors including the user's activity, heart rate, sleep, and diet, the medical information including the user's electronic medical record (EMR), family history, genetic vulnerability, and genetic susceptibility, and the digital therapeutics literacy including the user's accessibility, and technology adoption to the digital therapeutics and the apparatus.

In some embodiments, the outcome collection unit collects the execution outcomes of the digital instructions by monitoring the user's adherence to the digital instructions or allowing the user to directly input the user's adherence to the digital instructions.

In some embodiments, the generation of the digital instructions at the digital instruction generation unit and the collection of the user's execution outcomes of the digital instructions at the outcome collection unit are repeatedly executed several times with multiple feedback loops. In additional embodiments, the digital instruction generation unit generates the user's digital instructions for this cycle based on the user's digital instructions in the previous cycle and the execution outcome data on the user's digital instructions in the previous cycle collected at the outcome collection unit.

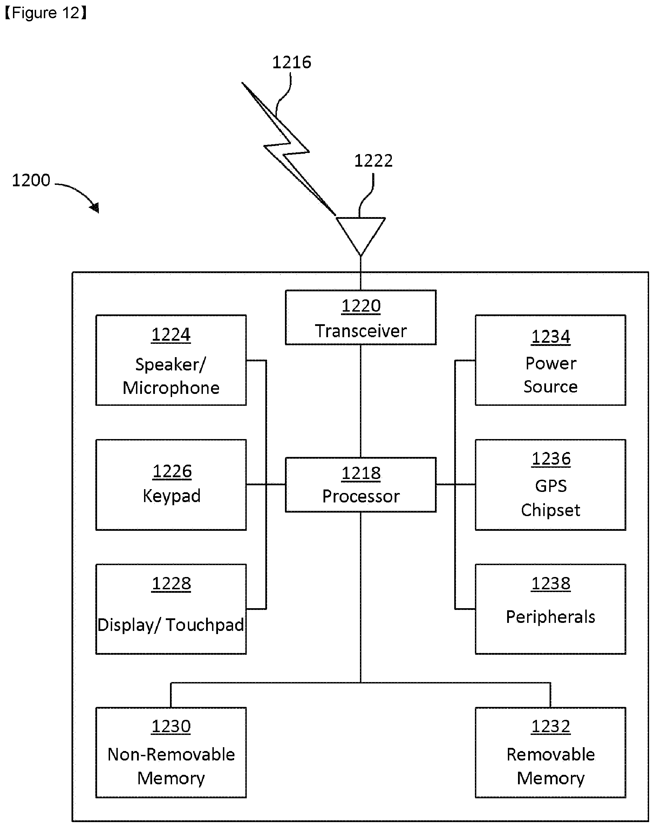

FIG. 12 illustrates an example device 1200 that can be used for performance enhancing application (PEA), which may be used in combination with any of other embodiments described herein. As shown in FIG. 12, the device 1200 may include a processor 1218, a transceiver 1220, a transmit/receive element 1222, a speaker/microphone 1224, a keypad 1226, a display/touchpad 1228, non-removable memory 1230, removable memory 1232, a power source 1234, a global positioning system (GPS) chipset 1236, and/or other peripherals 1238, among others. It will be appreciated that the device 1200 may include any sub-combination of the foregoing elements while remaining consistent with an embodiment. By way of example, the device 1200 may include a mobile device, a user equipment (UE), a mobile station, a fixed or mobile subscriber unit, a subscription-based unit, a pager, a cellular telephone, a personal digital assistant (PDA), a smartphone, a laptop, a netbook, a personal computer, a wireless sensor, a hotspot or Mi-Fi device, an Internet of Things (IoT) device, a watch or other wearable, a head-mounted display (HMD), a vehicle, a drone, a medical device and applications (e.g., remote surgery), an industrial device and applications (e.g., a robot and/or other wireless devices operating in an industrial and/or an automated processing chain contexts), a consumer electronics device, a device operating on commercial and/or industrial wireless networks, and the like.

The processor 1218 may be a general purpose processor, a special purpose processor, a conventional processor, a digital signal processor (DSP), a plurality of microprocessors, one or more microprocessors in association with a DSP core, a controller, a microcontroller, Application Specific Integrated Circuits (ASICs), Field Programmable Gate Arrays (FPGAs), any other type of integrated circuit (IC), a state machine, and the like. The processor 1218 may perform data processing, power control, input/output processing, sensor date processing, and/or any other functionality that enables the device 1200 to provide antiviral digital vaccine. The processor 1218 may be coupled to the transceiver 1220, which may be coupled to the transmit/receive element 1222. While FIG. 12 depicts the processor 1218 and the transceiver 1220 as separate components, it will be appreciated that the processor 1218 and the transceiver 1220 may be integrated together in an electronic package or chip.

The transmit/receive element 1222 may be configured to transmit data to, or receive data from a sever located in a medical institution or institution that manages the performance enhancing application (PEA). For example, instructions from a doctor or a coach and medical information sensed from a user may be received/transmitted from/to the server, via a base station over the air interface 1216. In one embodiment, the transmit/receive element 1222 may be an antenna configured to transmit and/or receive RF signals. In an embodiment, the transmit/receive element 1222 may be an emitter/detector configured to transmit and/or receive IR, UV, or visible light signals, for example. In yet another embodiment, the transmit/receive element 1222 may be configured to transmit and/or receive both RF and light signals. It will be appreciated that the transmit/receive element 1222 may be configured to transmit and/or receive any combination of wireless signals. The transceiver 1220 may be configured to modulate the signals that are to be transmitted by the transmit/receive element 1222 and to demodulate the signals that are received by the transmit/receive element 1222.

The processor 1218 of the device 1200 may be coupled to, and may receive user input data from, the speaker/microphone 1224, the keypad 1226, the display/touchpad 1228 (e.g., a liquid crystal display (LCD) display unit or organic light-emitting diode (OLED) display unit) and/or the peripherals 1238 (e.g., sensors or digital camera). The processor 1218 may also output user data or digital instructions to the speaker/microphone 1224, the keypad 1226, the display/touchpad 1228 and/or the peripherals 1238. In addition, the processor 1218 may access information from, and store data in, any type of suitable memory, such as the non-removable memory 1230 and/or the removable memory 1232. The non-removable memory 1230 may include random-access memory (RAM), read-only memory (ROM), a hard disk, or any other type of memory storage device. The removable memory 1232 may include a subscriber identity module (SIM) card, a memory stick, a secure digital (SD) memory card, and the like. In other embodiments, the processor 218 may access information from, and store data in, memory that is not physically located on the device 1200, such as on a server or a home computer (not shown).

The processor 1218 may receive power from the power source 1234, and may be configured to distribute and/or control the power to the other components in the device 1200. The power source 1234 may be any suitable device for powering the device 1200. For example, the power source 1234 may include one or more dry cell batteries (e.g., nickel-cadmium (NiCd), nickel-zinc (NiZn), nickel metal hydride (NiMH), lithium-ion (Li-ion), etc.), solar cells, fuel cells, and the like.

The processor 1218 may also be coupled to the GPS chipset 1236, which may be configured to provide location information (e.g., longitude and latitude) regarding the current location of the device 1200. In addition to, or in lieu of, the information from the GPS chipset 1236, the device 1200 may receive location information over the air interface 1216 from a base station and/or determine its location based on the timing of the signals being received from two or more nearby base stations. It will be appreciated that the device 1200 may acquire location information by way of any suitable location-determination method while remaining consistent with an embodiment.

The processor 1218 may further be coupled to other peripherals 1238, which may include one or more software and/or hardware modules that provide additional features, functionality and/or wired or wireless connectivity. For example, the peripherals 1238 may include an accelerometer, an e-compass, a satellite transceiver, a digital camera (for photographs and/or video), a universal serial bus (USB) port, a vibration device, a television transceiver, a hands free headset, a Bluetooth^{®} module, a frequency modulated (FM) radio unit, a digital music player, a media player, a video game player module, an Internet browser, a Virtual Reality and/or Augmented Reality (VR/AR) device, an activity tracker, and the like. The peripherals 1238 may include one or more sensors. The sensors may be one or more of a gyroscope, an accelerometer, a hall effect sensor, a magnetometer, an orientation sensor, a proximity sensor, a temperature sensor, a time sensor; a geolocation sensor, an altimeter, a light sensor, a touch sensor, a magnetometer, a barometer, a gesture sensor, a biometric sensor, a humidity sensor and the like.

In another aspect, the present disclosure relates to a computing system for enhancing performance of a user described herein, comprising: a display configured to provide, to the user, one or more first modules selected from the group consisting of a concentration module I for inducing dopamine secretion, an anxiety and/or stress module, a concentration module II for inducing melatonin secretion, and a coordination and agility module, each of the one or more first modules comprising one or more first instructions for the user to follow as disclosed above; a sensor described herein configured to sense adherence by the user to the instructions of the one or more first modules; a transmitter configured to transmit adherence information based on the adherence, to a server; and a receiver configured to receive, from the server, one or more second instructions based on the adherence information. In some embodiments, the display is further configured to provide, to the user, one or more second modules selected from the group consisting of a concentration module I for inducing dopamine secretion, an anxiety and/or stress module, a concentration module II for inducing melatonin secretion, and a coordination and agility module, each of the one or more second modules comprising the one or more second instructions.

In some embodiments, the digital application for enhancing performance instructs a processor of the digital apparatus to execute operations comprising: generating digital therapeutic modules for enhancing performance based on a mechanism of action in and a therapeutic hypothesis for enhancing performance. In some embodiments, the generating of the digital therapeutic modules comprises generating the digital therapeutic modules based on biochemical factors related to the performance enhancement.

In another aspect, the present disclosure relates to a non-transitory computer readable medium having stored thereon software instructions for enhancing performance of the user described above that, when executed by a processor, cause the processor to: display, by an electronic device to the user, the one or more first modules described above, each of the one or more first modules comprising instructions for the user to follow; sense, by the sensor described above in the electronic device, adherence by the user to the instructions of the one or more first modules; transmit, by the electronic device, adherence information based on the adherence, to a server; receive, from the server, one or more second instructions based on the adherence information; and display, to the user, the one or more second modules described above, the one or more second modules comprising the one or more second instructions.

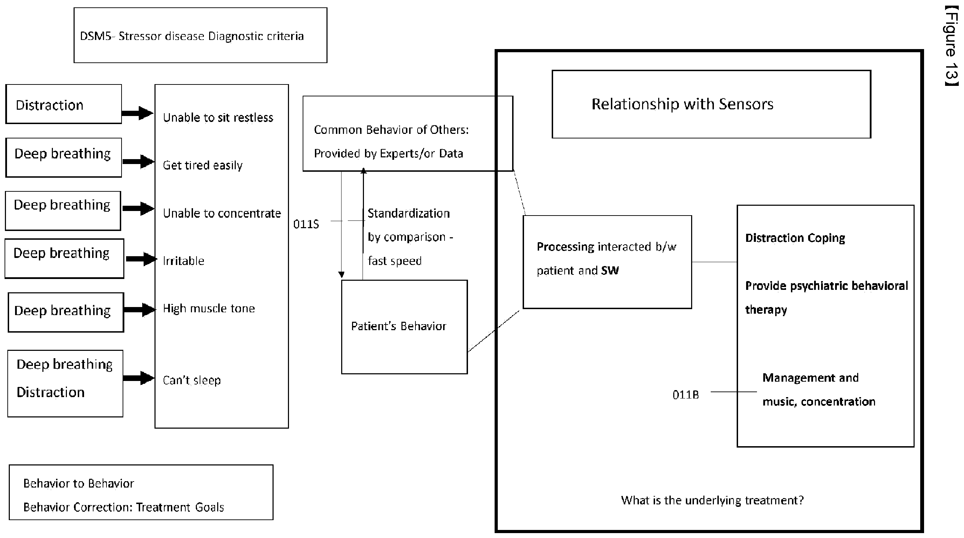

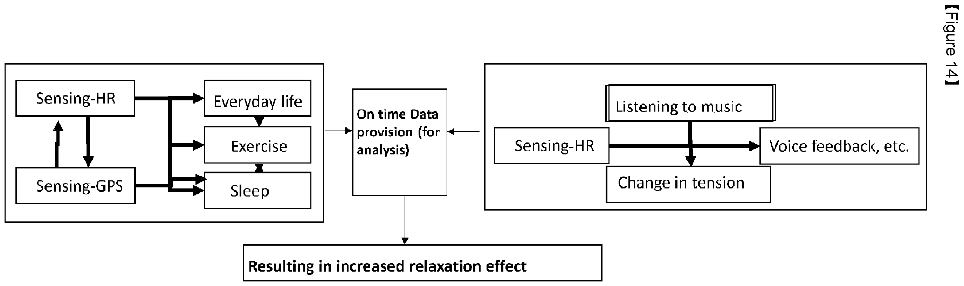

FIG. 13 is a diagram illustrating exemplary stressor disease diagnostic criteria for performance enhancing application (PEA) and relationship with sensors, and FIG. 14 is a diagram illustrating a data sensing standardization for performance enhancing application (PEA). It is impossible for humans to provide on-time prevention and treatment functions by connecting sensors and sensors (requires fast information processing function). Thus, an exemplary embodiment of the present disclosure enables adding the device technique to the sensing and preventing/treating patients. Referring to FIG. 13, in 011S, a motion capture can be used to collect individual movement data and standardize the movement of ski players in real time (a technique of standardizing 30 times per second by deep camera, for example), by standardizing to increase analysis clarity and showing results different from other related techniques. The data may be analyzed so that the standard playing input into the device or the playing can be corrected retrospectively by a coach, for example. It may be a device that can perform behavior correction on time, or by sending the behavior correction feedback of the athlete in real time. In some embodiments, the device may have a calibration function based on the real-time data from the athlete and/or external data, such as data from coach, already built-in data, and control data. The device may also provide feedback to an athlete by continuously calibrating the athlete's motions and analyzing the motions in real time compared to the past performances. In 011b, data can be processed through HR sensor data and a SW algorithm interlocking GPS (how quickly and accurately quantify recognition), and the processed data can be provided to the designated personals (e.g., skiers) on time, as illustrated in FIG. 14. In some embodiments, 011b is the end point of the whole system, that is, the part that ultimately manages the mental (relieving anxiety and tension, and giving the effect of concentration) in the device. Here, it may be a device to help instantly concentrate and relax in real time when the heart rate does not stabilize by using voice or other methods to stabilize the heart rate (e.g. deep breathing, listening to music, or thinking about something good) while sensing the heart rate more precisely in real time.

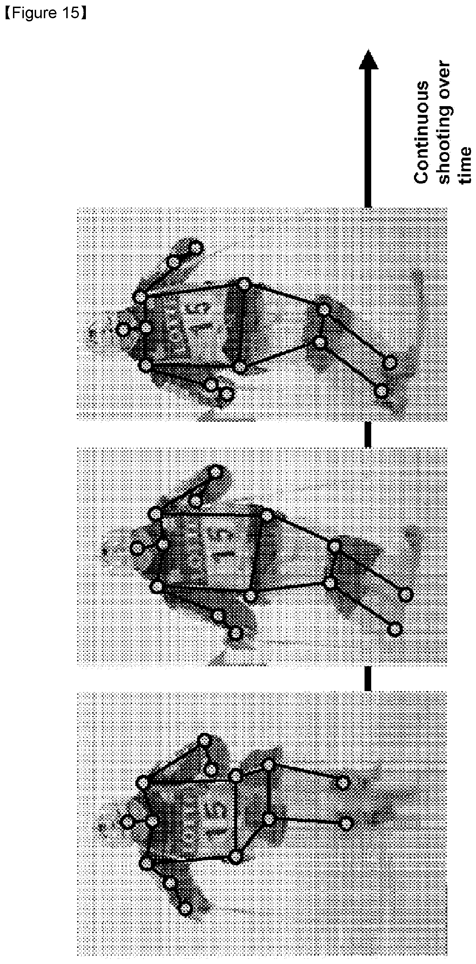

FIG. 15 is a diagram illustrating an exemplary physical part analysis using motion detection. For example, each body part can be analyzed, such as close stance of both knees and ankles, movement of the pelvis, an upper body lower back, neck shake, wrist movement, etc., to analyze the posture of the skier using motion detection.

In further embodiments, when the user is a skier, the sensor described herein detects positions of knees and/or ankles of the skier, and the distances thereof. In further embodiments, the sensor described herein detects positions of the neck, shoulder, arms and/or hip of the skier.

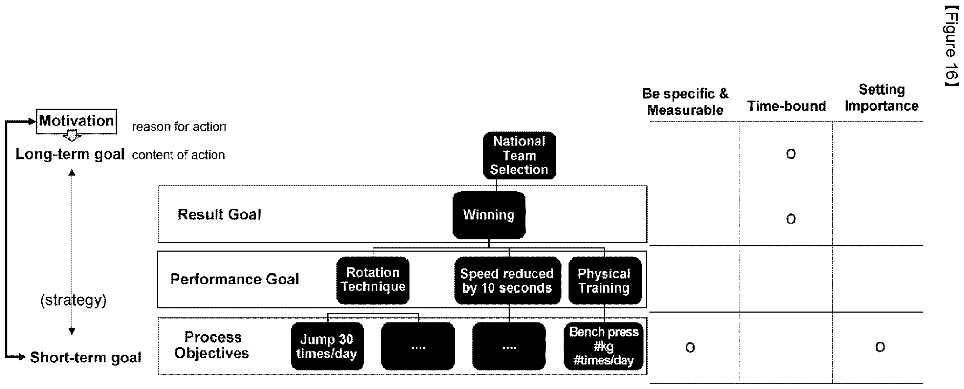

FIG. 16 is a diagram illustrating an exemplary process of setting goals for athletes using performance enhancing application (PEA). A goal setting is important in planning how players think, feel and behave, and it is important to set effective and correct goals. According to the present disclosure, the application may provide a way to provide information when setting goals without user-input, and it is possible to modify the goal setting in the event of an injury, slump, failure, etc. The application may further provide necessity and method and may include information such as evaluation frequency, evaluation contents (why the goal is achieved or not achieved, records of assessments and advice from leaders, etc.). Such a goal setting can be the driving force to achieve the set goals. The user (e.g., skiers) can check whenever motivation is necessary.

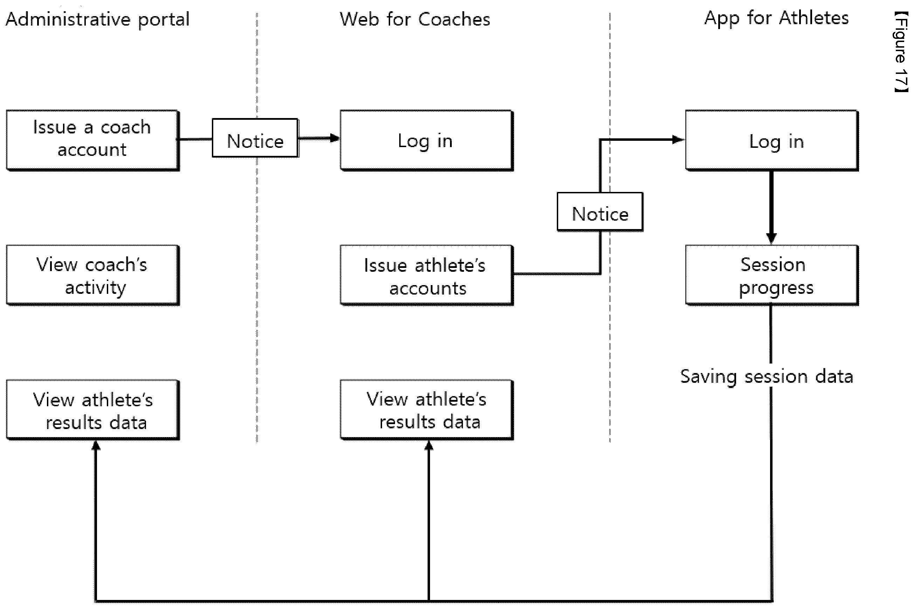

FIG. 17 illustrates a system including a performance enhancement application (PEA), wherein the system includes an administrative portal (e.g., an administrator's web), a portal for coaches (e.g., a coach's web), and a digital apparatus configured to execute a digital application (e.g., an application or "app") for enhancing the performance of the user.

In one embodiment, the system may enable an administrator to issue coach accounts, view coach activity information, and view information related to athletes via the administrative portal. In one embodiment, the system can enable an exercise scheduling provider (e.g., a coach) to issue an athlete account via the portal for coaches, and review athlete information (e.g., age, athlete's condition, athlete's scheduling information, game schedule, completion status of one or more modules or sessions (e.g., a concentration module, an anxiety module, a stress module, a coordination module, an agility module, etc.)) In one embodiment, the system may enable the athlete to complete the one or more modules or sessions via a digital application. In one embodiment, the system may allow the coach or administrator to view result data of performing the one or more modules or sessions by the athlete. The coach or administrator can view the athlete's result data and modify the one or more modules or sessions that the athlete is scheduled to complete.

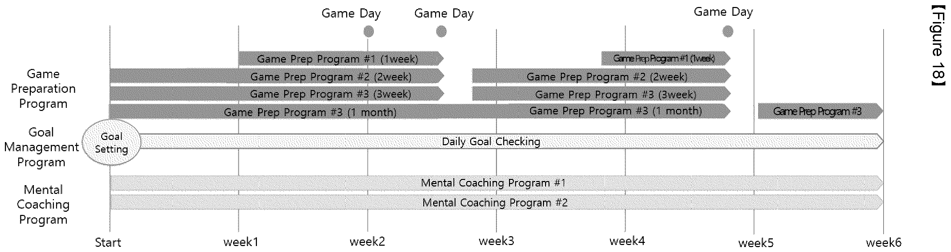

FIG. 18 illustrates an example timeline of programs that may be provided through a performance enhancement application (PEA). In one embodiment, the programs provided through the performance enhancement application (PEA) may include a game preparation program, a goal management program, and a mental coaching program. The game preparation program may refer to a training program for enhancing an athlete's performance over time, such that the athlete performs at his or her best on a set game day. For example, the game preparation program may provide a training schedule from one month prior to a set game date to the day of the game date. In one embodiment, if multiple games are set, the game preparation program may provide a training schedule based on the closest game. The goal management program may refer to a training program provided to achieve a set goal. For example, the goal management program may provide a schedule that a user should perform daily or periodically to achieve a set goal. Alternatively, the goal management program may provide a schedule of games that the user must win to achieve the set goal, training related to qualifications that the user must meet to compete in a particular game, etc. The mental coaching program may refer to a program provided to strengthen the user's mental component. For example, the mental coaching program may include meditation, muscle stretching, motivation, image training, listening to music, etc. In one embodiment, meditation during the mental coaching program may mean having the user watch a meditation video for five minutes to calm the user's mind and body, and muscle stretching during the mental coaching program may mean having the user perform a prescribed stretch, motivation during the mental coaching program may mean having the user write a motivational sentence, image training during a mental coaching program may mean having the user view a photo or video and imagine a positive image, and listening to music during the mental coaching program may mean having the user listen to music that helps the user calm the mind and body. The game preparation program, goal management program, and mental coaching program described above may be provided to the user simultaneously. Furthermore, the game preparation programs, goal management programs, and mental coaching programs described above are by way of example only and do not limit the rights of the present disclosure.

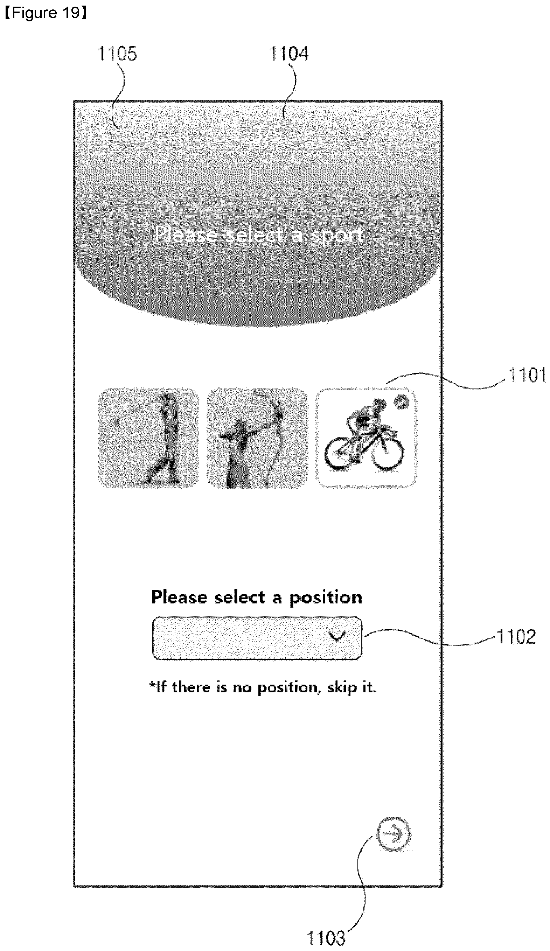

Next, FIGS. 19 to 38 illustrate examples of screens of a performance enhancement application (PEA) provided to a user. The performance enhancement application (PEA) may be provided to the user via the device 1200 described in FIG. 12.

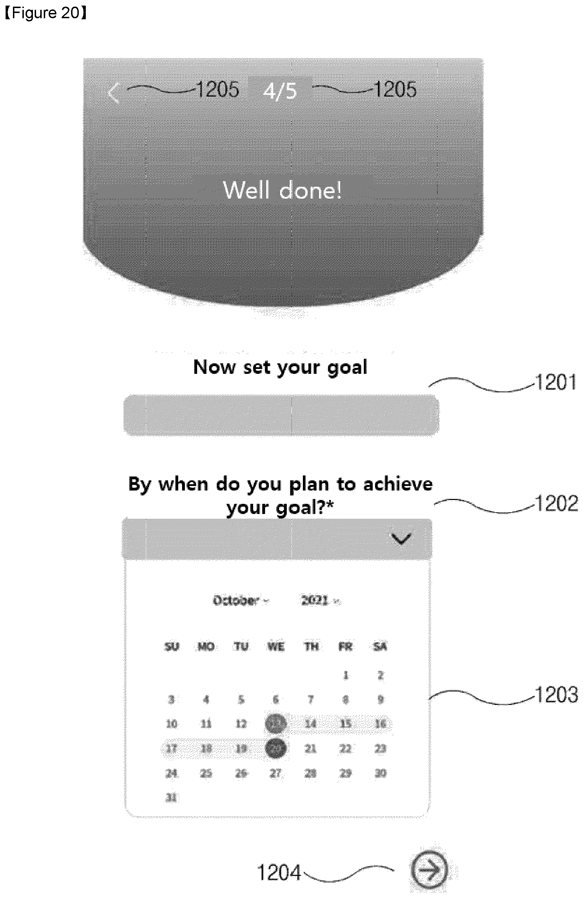

FIG. 19 illustrates an sport setting screen of a performance enhancement application (PEA) of the present disclosure. In one embodiment, the user may select a sport via a sport selection icon 1101. For example, the user may select the sport for which the user wishes to train, such as golf, archery, cycling, or the like, from a list of different types of sports. After selecting the sport, the user can select a position within the sport by clicking the position selection menu 1102. For example, if the game is baseball, the user can select the position they wish to train for, such as pitcher or batter. The performance enhancement application (PEA) may provide a drop-down menu of position types for the user to select a position. If the user selects a sport that does not have positions, the position selection menu may not be enabled, and the user may not select a position. When the user clicks the previous button 1105, the screen of the application returns to the initial setup screen, and when the user clicks the next button 1103, the screen of the application advances to the setup screen of FIG. 20. In one embodiment, the top of the screen may display a page number 1104.

FIG. 20 illustrates a goal setting screen of a performance enhancement application (PEA). In one embodiment, a user can enter his or her goal in the goal setting bar 1201. For example, in the goal setting bar 1201, the user may enter a goal of winning a game A, being selected for a national team, etc. Then, when the user clicks the goal achievement date selection button 1202, a calendar 1203 may be displayed. From the calendar 1203, the user can select a date to accomplish the goal. If the user clicks the previous button 1206, the application screen may return to the sport selection screen of FIG. 19, and if the user clicks the next button 1204, the application screen may proceed to the exercise start date setting screen. If the user does not set a exercise start date, the exercise start date may be set to the same day as the date the user set his or her goal. In one embodiment, the top of the screen may display a page number 1205.

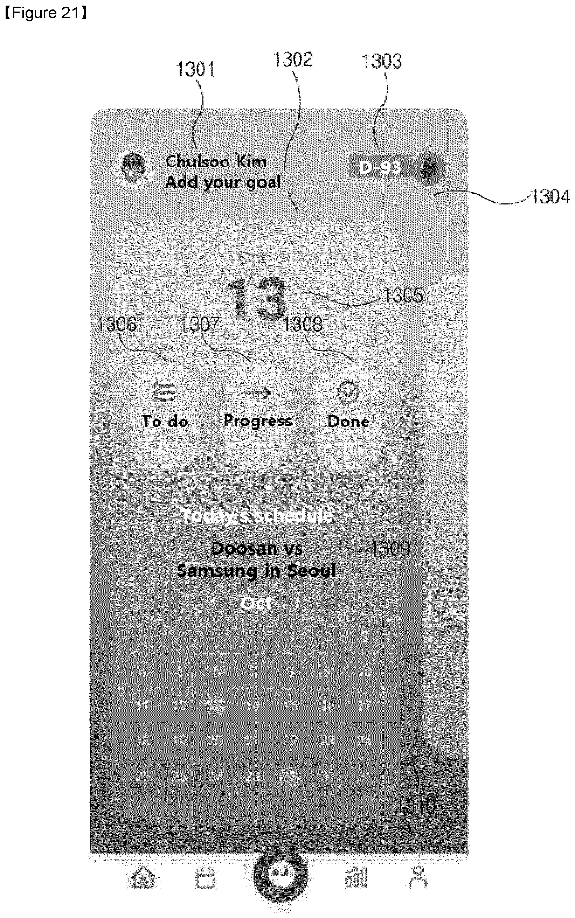

FIG. 21 illustrates a main screen of a performance enhancement application (PEA). The top of the screen may display an athlete's name 1301, a goal 1302, days remaining to the goal achievement 1303, and an icon 1304 indicating a wearable device connection. If the user taps the goal 1302 icon, the user can go to the user's My Page screen. The icon 1304 indicating the wearable device connection may indicate whether the wearable device is connected to the device, the type of wearable device connected, etc. If a wearable device is connected to the device, the performance enhancement application (PEA) may collect sleep information (e.g., sleep duration, sleep state, sleep efficiency, sleep patterns, etc.), exercise information (e.g., exercise duration, exercise state, calories burned, exercise patterns, etc.), and heart rate information (e.g., heart rate changes when performing a training program, etc.) from the user via the wearable device and transmit the information to an external device. Then, the main screen may display a selected date icon 1305, a program 1306 that should have taken place today, a program 1307 that is in progress today, and a program 1308 that was completed today. Below that, a schedule 1309 may be displayed, which may be today's schedule or a schedule that exists on the selected day in the calendar 1310 at the bottom. In this case, if there is a game scheduled for the selected date, the name of the game may be displayed, as shown in FIG. 21, and if there is no game scheduled, the number of programs that need to take place on that date may be displayed. The user may select a date on the calendar 1310 to view the schedule. The user can switch between screens by swiping across the screen from the main screen.

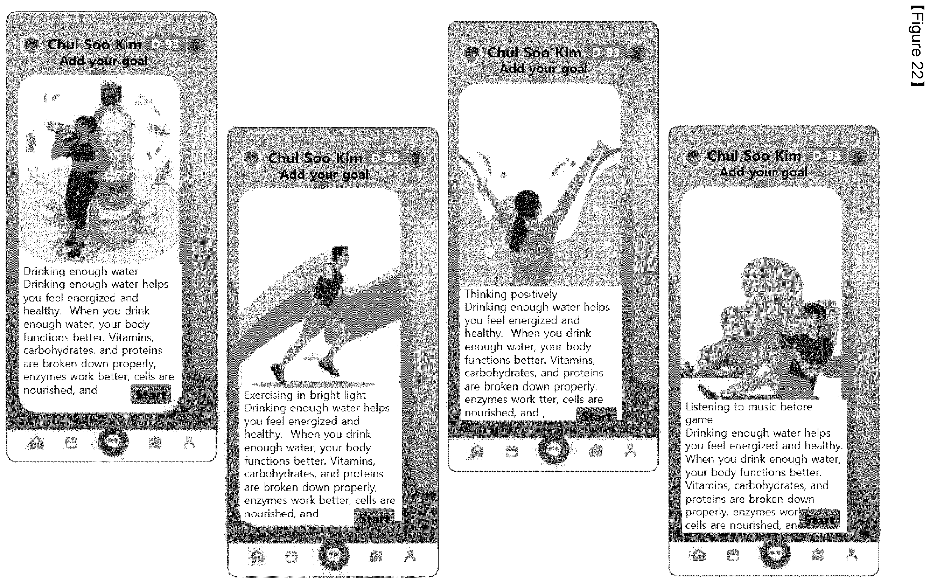

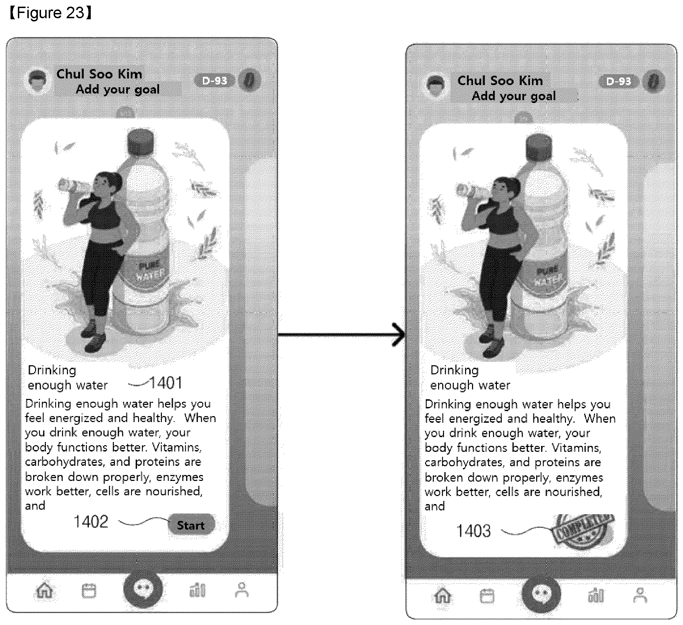

Next, FIG. 22 illustrates examples of screens of a game preparation program. The performance enhancement application (PEA) may provide different preparation programs based on the sport and position selected by the user. The game preparation program may instruct the user to perform actions such as "drinking enough water", "exercising in bright light", "thinking positively", and "listening to music before game". FIG. 23 illustrates an example of screen when a user performs a game preparation program. For example, if the game preparation program is "Drinking enough water," the program title 1401 may display "Drinking enough water," and a description of the program may be displayed at the bottom of the title. After the user reads the description, and before or after clicking the start button 1402, an indication 1403 that the user has completed the program may appear, and the performance enhancement application (PEA) may record the user's completion of the program on the device.

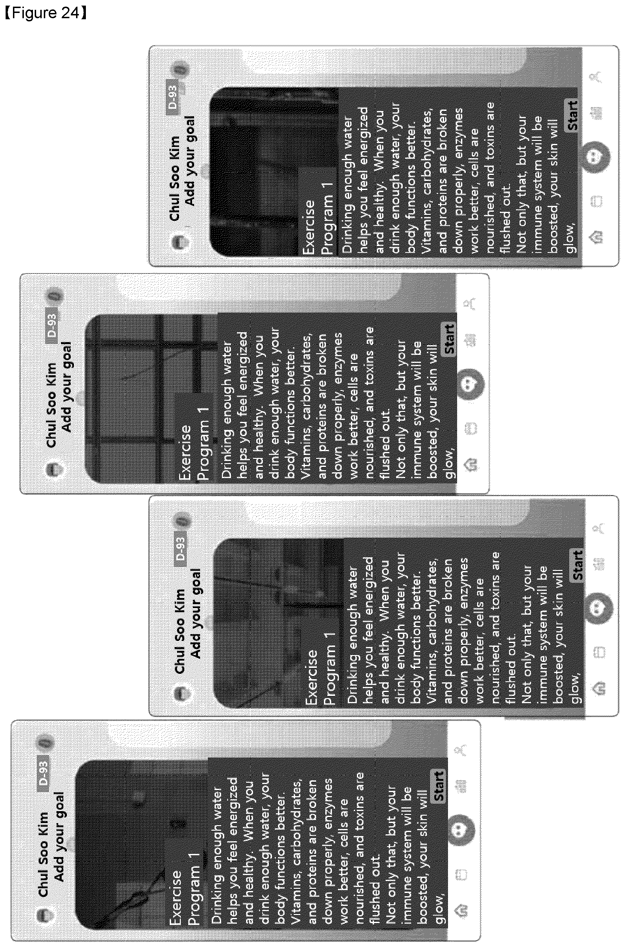

FIG. 24 illustrates exemplary screens of a goal management program. The performance enhancement application (PEA) may provide different goal management programs based on the sport, position, and goal selected by the user. The goal management program may direct the user to perform a specific exercise or exercise routine.

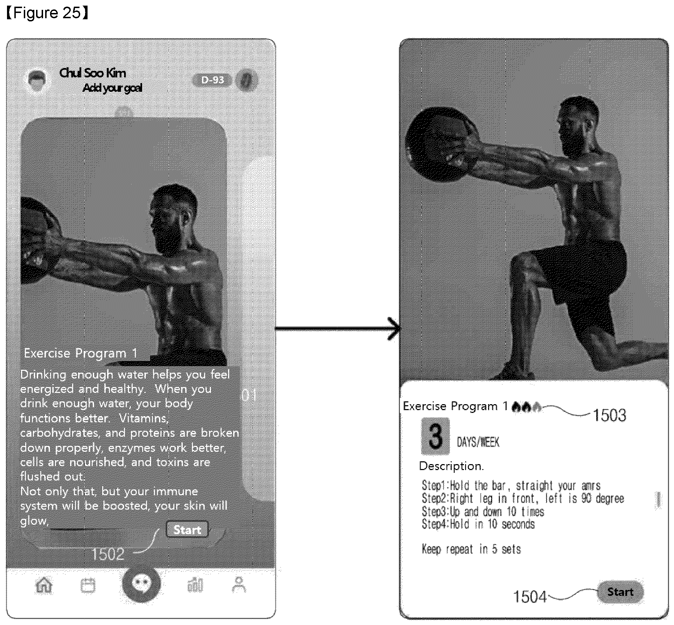

FIG. 25 illustrates an example of screen when a user performs a goal management program. For example, the goal management program may provide a period and training frequency to achieve the goal. A program title 1501 may display a program name and difficulty level, and a description of the program may be displayed at the bottom of the title. After the user reads the description, but before clicking the start button 1502, the exercise instructions 1503 included in the program may appear. If the wearable device is connected to the device 1200 executing the performance enhancement application (PEA), when the user clicks the start button 1504, the processor 1218 of the device 1200 may receive data (e.g., heart rate data) from a sensor on the wearable device and use the received data to determine whether the user is performing the exercise. When the processor 1218 of the device 1200 executing the performance enhancement application (PEA) determines that the user has performed the exercise, the processor 1218 may record that the program is complete. Additionally, when the device 1200 records the completion of the program, the device 1200 may record data received from the sensors of the wearable device.

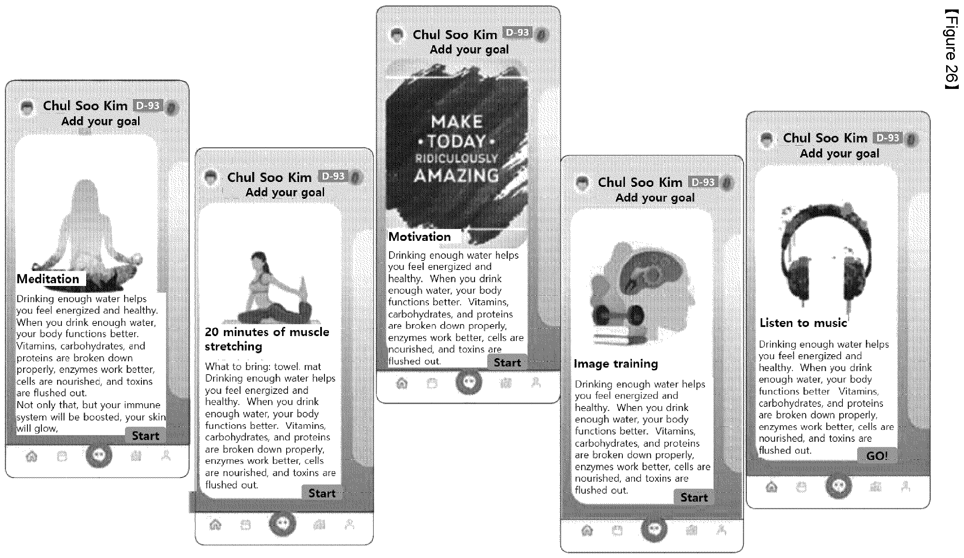

FIG. 26 illustrates example screens of a mental coaching program. The performance enhancement application (PEA) can provide meditation, muscle stretching, motivation, image training, listening to music, etc. through the mental coaching program.

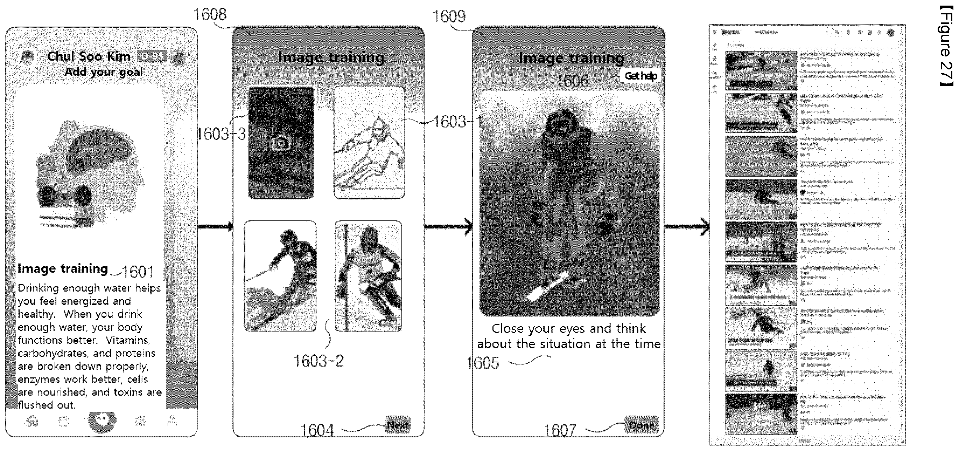

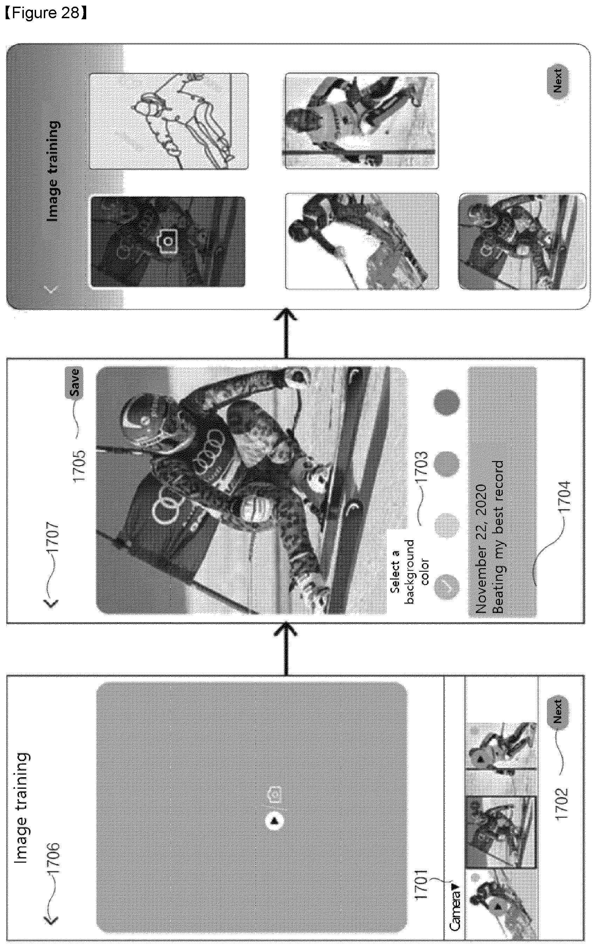

FIG. 27 illustrates example screens of image training in a mental health program. If the mental health program is "Image training," the program title 1501 may display "Image training," and a description of the program may be displayed at the bottom of the title. When the user reads the description and clicks the start button 1502 to do so, a screen may appear to select a photo for image training. The user may select one of the default template 1603-1, an image pre-uploaded by the user 1603-2, or a new icon 1603-3, and click the next button 1604. When the user selects the default template 1603-1 and clicks the next button 1604, a photo and description 1605 for image training may be displayed on the screen. The photo and description 1605 for image training may vary depending on the sport and position selected by the user. If the user clicks the 'Get help' button 1606 located at the top of the screen, a list of videos related to image training for that sport may be displayed. If the user clicks the previous buttons 1508, 1509, the application returns to the previous settings screen. If the user selects the new icon 1603-3 and clicks the next button 1604, the screens shown in FIG. 28 may be displayed.

FIG. 28 illustrates an example of screen for creating a new image training screen in a mental coaching program. A photo or video 1701 may be displayed at the bottom of the screen. The photo or video 1701 displayed at the bottom of the screen may be a photo or video stored in memory or may be a photo or video provided by an external device. The photo or video 1701 may be a result of the application learning which photos or videos the user frequently uses and providing as their similar content. The user may select one of the photos or videos 1701. The user selects one of the photos or videos 1701 and taps the next button 1702 to move to the next screen. The user can write something in the notes window 1704 to help train the image and use the color picker icon 1703 to color the notes window 1704. The user can save the new image training screen by tapping the save button 1705, and once the screen is saved, the user can select the created image training screen from the selection screen. When the user taps the previous buttons 1706, 1707, the application returns to the previous screen.

Although not shown in the FIGs, in one embodiment, the performance enhancement application (PEA) can analyze a user's body (e.g., posture, angle, etc.) to provide content that includes ideal postures, angles, or movements when the content uploaded for image training is a photo or video of the user's own body. In another embodiment, the performance enhancement application (PEA) can search for and recommend content similar to the uploaded content when the uploaded content for image training is a photo or video of someone other than the user. For example, the application can analyze and identify the person in the uploaded content, and search for and serve content of the identified person. Alternatively, the application may search for and provide game (training) content from athletes other than the identified person. When providing relevant content, the application may first consider the sport selected by the user to shorten the search process. In addition, the application may analyze the total number of views of the content, the awards of the athlete in the content, and information about the athlete when providing relevant content, and may serve the content to the user based on the analysis.

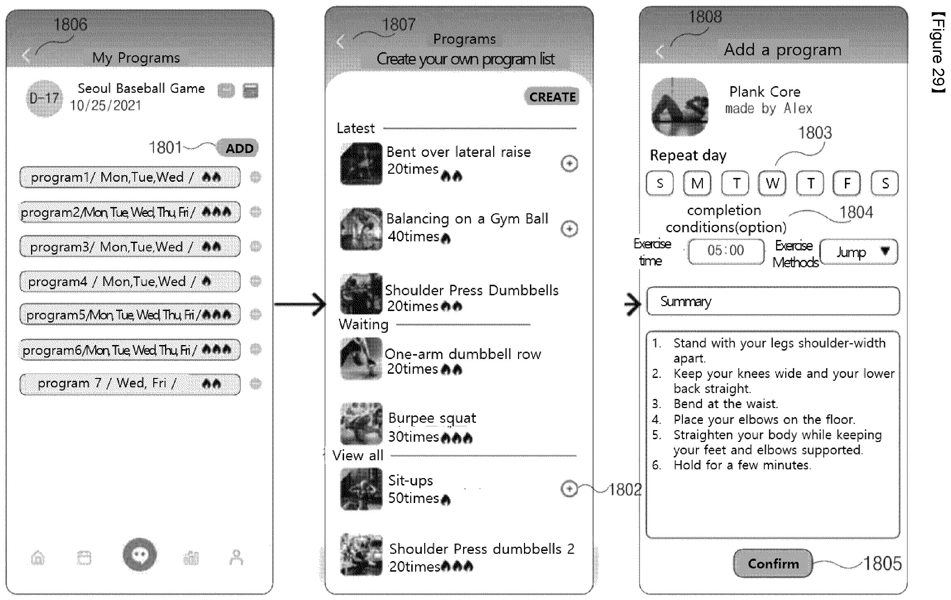

Next, the user may add or create his or her own training program through the screens of FIGS. 29 to 33. FIG. 29 illustrates exemplary screens for the user to add a new training program. When the user taps the add program button 1801, the screen of the performance enhancement application (PEA) switches to the add program screen. The 'Add a program' screen may display different recommended programs based on the sport or position the user is enrolled in. Also, if the user is a member of a specific team, the training programs set up by the team may be displayed. If the user taps the plus (+) button 1802 next to a program they wish to add from the displayed programs, the Performance Enhancement Application (PEA) screen switches to the detailed settings of the program to be added. If the user selects an exercise named "Plank Core," the screen switches to the detailed settings screen for the "Plank Core" program, and the user can select a day of the week 1803 to repeat "Plank Core" on the detailed settings screen. At this point, the user may select a completion condition 1804. The completion condition 1804 may only be set when the device 1200 is associated with a wearable device, and the completion condition 1804 may comprise a time to perform, an exercise modality, etc. In one embodiment, The completion condition 1804 may be set on the condition that the user's heart rate detected through the wearable device is maintained for a predetermined time or longer. When the user selects the repeat day and completion condition, and taps the OK button 1805, the program is added and the screen may switch to the program list screen. When the user clicks the previous buttons 1806, 1807, 1808, the application returns to the previous screen.

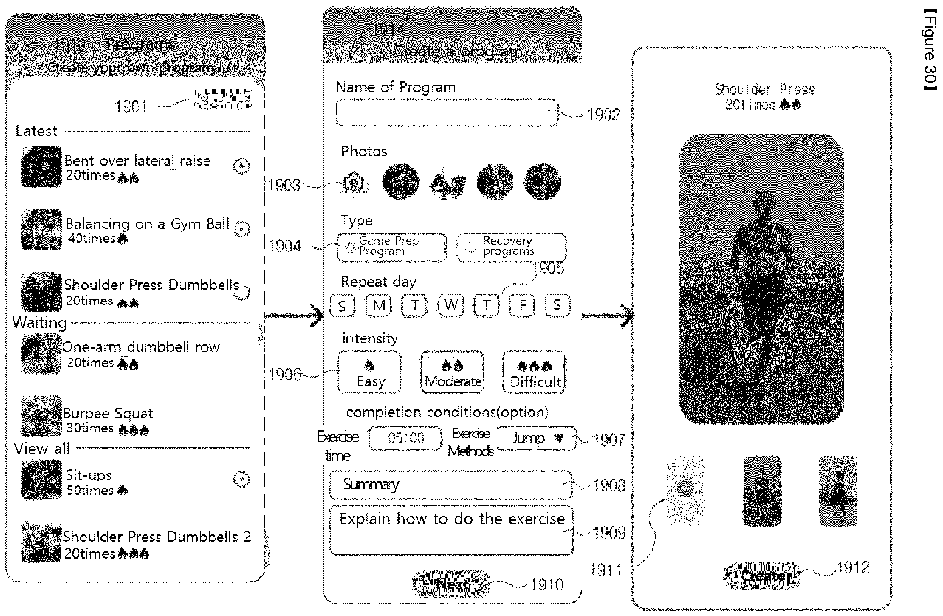

FIG. 30 illustrates example screens for a user to create a new training program. When the user taps the 'create a program' button 1901, the screen of the performance enhancement application (PEA) switches to the program creation screen. The user can enter a name for the program to be created in the program name input field 1902 and select a program picture 1903. The program picture 1903 can be a picture stored in memory or an externally supplied picture. The user can select a program type 1904 and select a repeat day of the week 1905. The selected repeat days 1905 may be modified when adding training programs as described above. The user may select a training intensity 1906, and may select a completion condition 1907. The user can enter a short description of the training program in a short description input window 1908, and a detailed exercise method in an exercise method input window 1909. When the user clicks the next button 1910, the screen switches to a screen for selecting a card photo. The user can select a photo 1911 to be used when displaying the program on the main screen. When the user taps the Create button 1912, the training program is created and the created program is displayed in the list of programs. When the user taps the previous buttons 1913, 1914, the application returns to the previous screen.

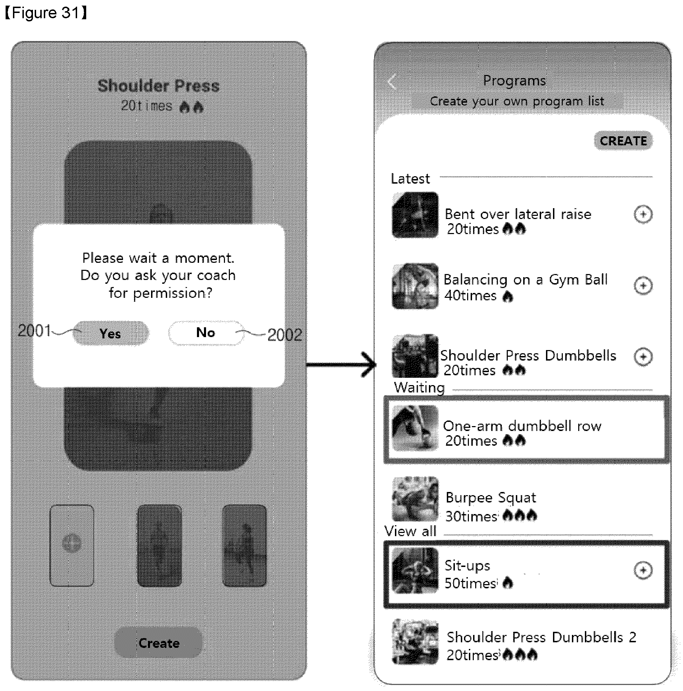

FIG. 31 illustrates an example of screen for requesting permission from a coach when a user has created a new training program. As in FIG. 30, when a user creates a training program, the screen of the performance enhancement application (PEA) may display a pop-up for requesting permission from the coach. If the user taps the Yes button 2001, the user-created program is placed in the waiting list and no training program can be added until the coach's permission is received. If the user taps the No button (2002) in the pop-up, the program created by the user is displayed in the 'View all' list and can be added to the training program without the coach's permission. The coach may receive a request for permission to approve a training program from an athlete via the web or app, and may send a signal to the athlete's device 1200 indicating permission or disapproval via the web or app.

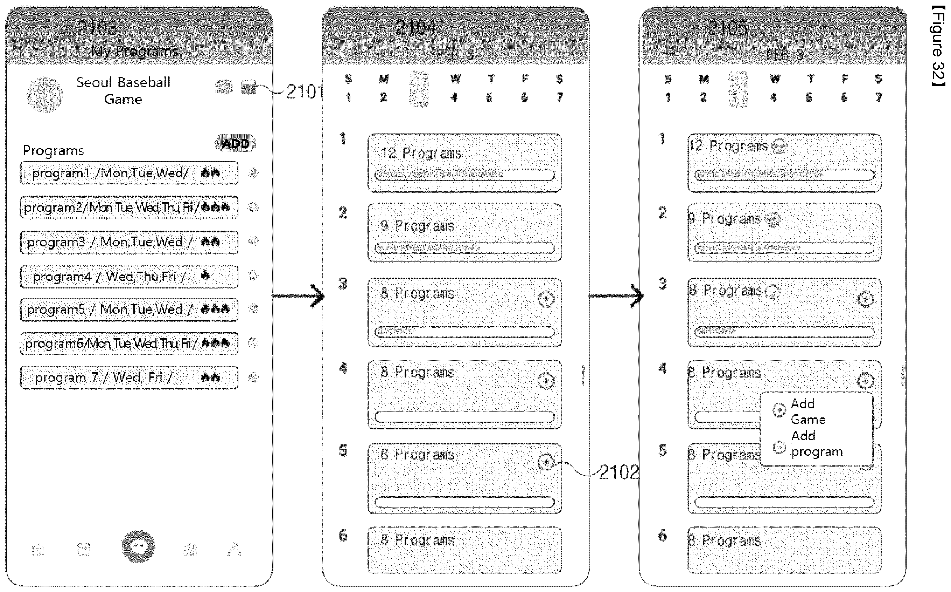

FIG. 32 illustrates example screens for a user to add a game or program on the schedule screen. When the user taps the calendar icon 2101 on the program list screen, the user can go to the schedule screen. The schedule screen may display programs that have been completed, programs that are in progress, or programs that need to be completed for a predetermined period of time (e.g., six days) before or after a specific date. The user may click the plus (+) button 2102 to add a game or program to a specific date. If the user is a player on a particular team, the user may be set to add a game or program only after receiving permission from the coach to add the game or program. When the user clicks the previous button 2103, 2104, 2105, the application returns to the previous screen.

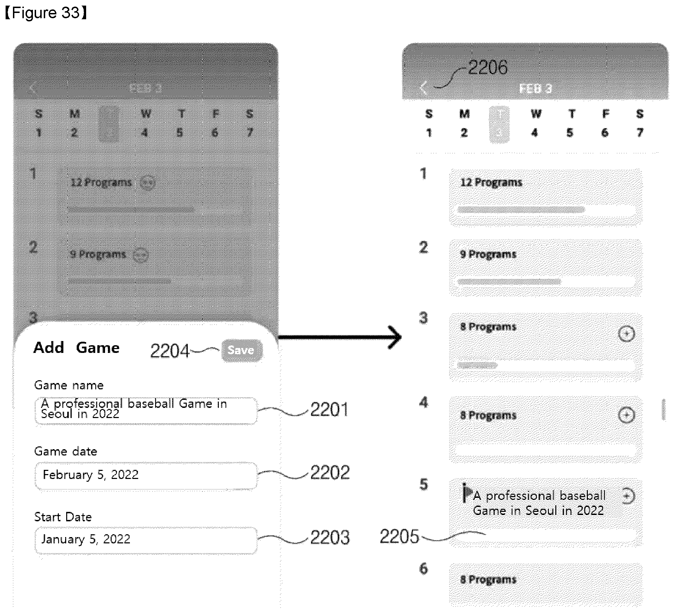

FIG. 33 illustrates an example of screen for a user to add a game from the Schedule screen. When the user taps the 'Add Game' button on the schedule screen, the Add Game window may be displayed on the screen. The user may enter a game name in the Game Name input window 2201, a game date in the Game Date input window 2202, and a training start date in the Start Date input window 2203. When the user taps the Save button 2204, the game is added and the added game 2205 is displayed in the schedule. When the user taps the previous button 2206, the application returns to the previous screen.

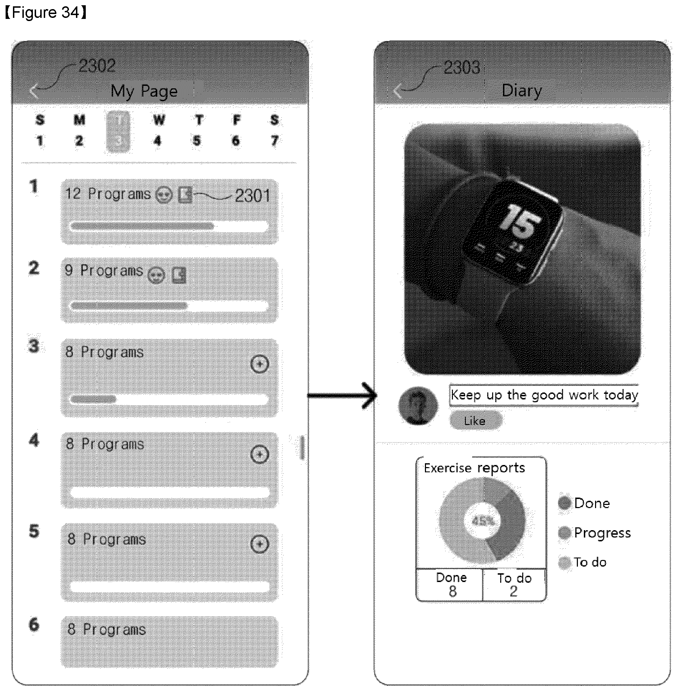

FIG. 34 illustrates an example of screen for a user to view a diary for a particular day on the schedule screen. When the user taps the diary icon 2301 on the schedule screen, the diary screen for that day may be displayed. On the diary screen, the user may record notes related to the day, and may view the training history for the day.

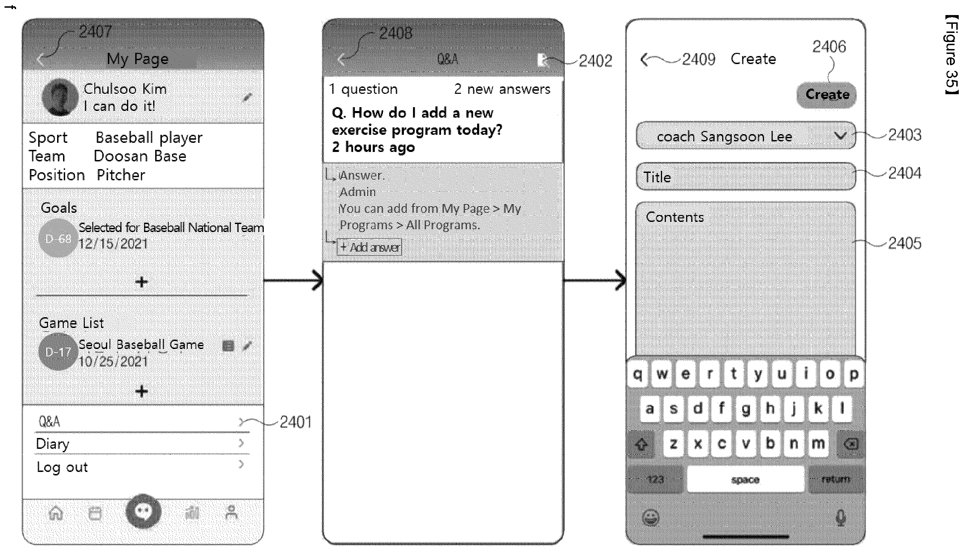

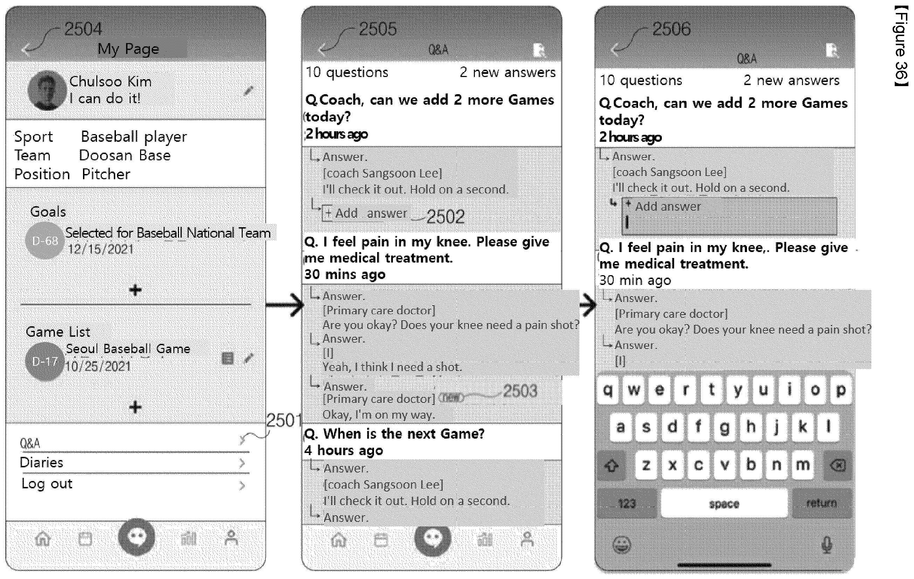

FIGS. 35 and 36 illustrate example screens for a user to communicate with an administrator via the performance enhancement application (PEA). When the user taps the Q&A button 2401 on the My Page, a Q&A list screen may be displayed. The Q&A list screen may display questions that the user (or a team the user is a member of) has left for the administrator. From the Q&A list screen, the user can tap the write button 2402 to switch to the Q&A creation screen. The user can enter the person to be questioned (i.e., the person to whom the question is directed (e.g., a coach, manager, or administrator)) in the input window of the person to be questioned 2403, and enter a question title and question content in the question title input window 2404 and question content input window 2405. When the user taps the Save button 2406, the question is added and the post is registered. Once the question is registered, the device 1200 executing the performance enhancement application (PEA) may send a signal to the selected person to be questioned to notify him or her that the question has been registered. When the user clicks the previous buttons 2407, 2408, 2409, the screen of the application returns to the previous screen. In the Q&A list screen of FIG. 36, the user can reply again to the administrator's answer to the Q&A by tapping the add answer button 2502. A new icon 2503 may be displayed next to the newly added answer to indicate that it is a newly added answer. The new icon 2503 can be set to disappear after a predetermined period of time after the answer is registered. When the user clicks the previous buttons 2504, 2505, 2506, the application returns to the previous screen.

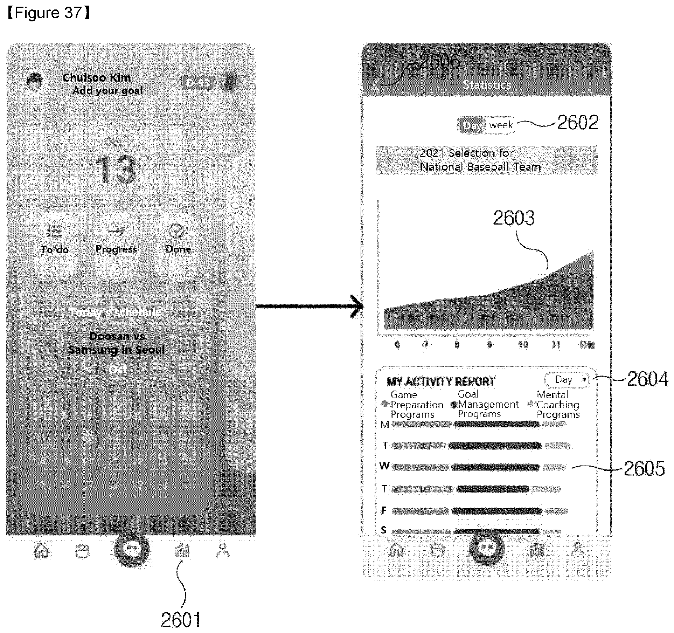

FIG. 37 illustrates an example of screen for a user to view program usage statistics. When the user taps the graph icon 2601 on the main screen, the screen may switch to the statistics screen. On the statistics screen, the user can tap the time period setting icon for the stacked graph 2602 to view training statistics in the form of a stacked graph on a daily or weekly basis. The statistics screen may display a stacked graph 2603 based on the selected units. The cumulative graph 2603 represents the user's accumulated exercises over a set period of time. The user can tap the duration setting icon for the program completion graph 2604 on the statistics screen to view training statistics in the form of a bar graph on a daily or weekly basis. The program completion graph 2605 shows how many programs the user has completed in the selected day or week in the form of a bar graph. When the user taps the previous button 2606, the application returns to the main screen.

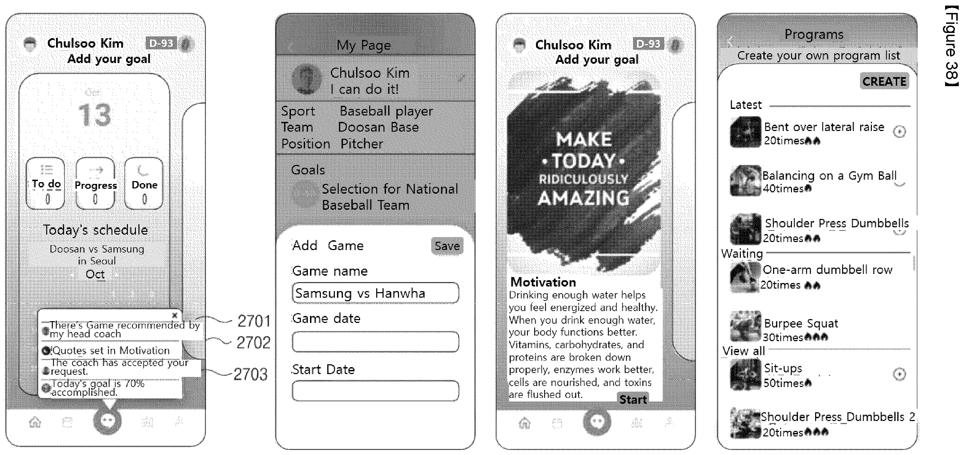

FIG. 38 illustrates examples of screens for a user to view notifications within a performance enhancement application (PEA). The main screen may display various notifications related to a training program. Notifications related to the training program may include a suggested game notification 2701, a quote notification 2702, a request acceptance notification 2703, and the like. When the coach recommends a game to the user to add to a training program, the user's application may display the recommended game notification 2701, and when the user taps the recommended game notification 2701, the screen may transition to the add game screen illustrated in FIG. 33. The user can simply click on the notification from the coach to add the game to their schedule. Additionally, if the user taps the quote notification 2702, the application may transition to a motivational screen. Finally, if the coach (manager) has approved a training program created by the user, the user's application may display a request acceptance notification 2703, and if the user taps the request acceptance notification 2703, the application may switch to the add program screen described above in FIG. 31.

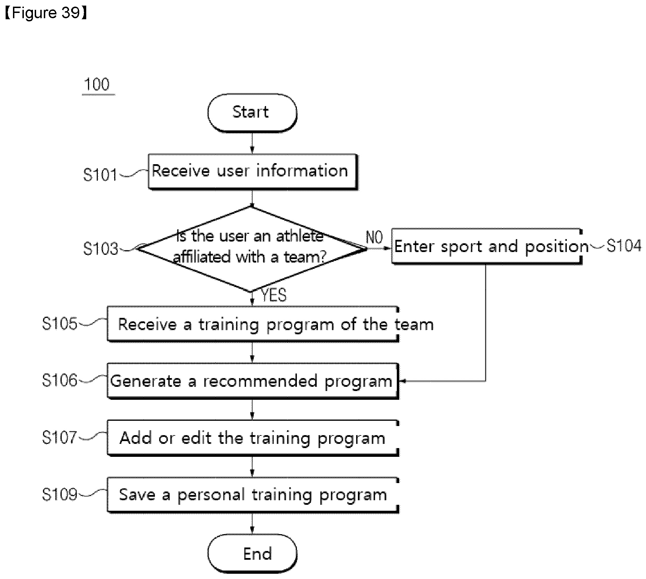

Next, FIGs. 39 to 41 illustrate exemplary flowcharts of processes 100, 200, 300 of a performance enhancement application (PEA) that may be performed by a processor according to disclosed embodiments. For example, the processes 100, 200, 300 may be performed in whole or in part by the device 1200 (e.g., using the processor 1218) of an athlete seeking to enhance performance. One of ordinary skill in the art will understand that the steps illustrated in FIGs. 39 through 41 are exemplary only, and that in some embodiments, steps may be added, merged, split, duplicated, repeated, modified, performed sequentially, performed in parallel, and/or deleted.

First, referring to FIG. 39, describe a method of operation of a device executing a performance enhancing application (PEA).

When the performance enhancement application (PEA) is launched, the device 1200 may receive input of information from a user attempting to utilize the application (S101). The input of user information may be an attempt by the user to log in to the application. When the user logs into an account through the application, the device 1200 may receive the user's information from the server. Further, the user information may include at least one of a user's goals, a game schedule, and the like. In one embodiment, if the user enters his or her goal, the device 1200 may provide a goal management program as a recommended program in step S106 described later, and if the user enters a game schedule, the device 1200 may provide a game preparation program as a recommended program in step S106.

The device 1200 receiving the user information may determine, based on the user information, whether the user is an athlete affiliated with a team (S103). If the user is an athlete affiliated with a team, the device 1200 may receive a training program for the team the user is affiliated with (S105). The device 1200 may request the training program of the team to which the user belongs from a server, and receive the training program transmitted from the server in response to the request.

If the user is an individual athlete who is not affiliated with a team, the device 1200 may receive input (S104) of at least one of a sport or a position for which the user wishes to train. The device 1200 may request a training program from a server that is appropriate for the sport entered by the user, and may receive a sport-specific training program sent from the server in response to the request.

The device 1200 may generate a recommendation program to improve the user's performance based on the user information (S106). For example, if the user is an athlete on a team, the device 1200 may receive the training program of the user's team and generate a recommendation program based on the training program of the team. The device 1200 may generate a goal management program as a recommended program if the user has entered their goals, or a game preparation program as a recommended program if the user has entered a game schedule. The device 1200 can present the generated recommendation program to the user.

The device 1200 may receive input to add or modify a training program (S107). The addition or modification of the training program may be made directly from the athlete, or may be made by a request received from a coach's (manager's) device. In one embodiment, the device 1200 may send a request for permission to add or modify a training program to an external device upon receiving input from a user to add or modify the user's training program. For example, if the user of the device 1200 is a team athlete and the training program is set to require a coach's (manager's) permission to add or modify the training program, the device 1200 may send the permission request to an external device that is the coach's (manager's) device. In this case, the device 1200 may only add or modify the training program if it receives a permission signal from the coach's device. Conversely, the device 1200 may first receive a request to add or modify a user's training program from an external device, and then add or modify the training program by receiving a response from the user of the device 1200. In other words, if the user is a team athlete, the coach may send a request to add or modify a specific athlete's training program via the device, and the athlete may accept or decline the request by viewing a notification from the device 1200. Through step 107, the training program may be adjusted differently for different athletes, and in some cases, step 107 may be omitted.

The device 1200 may store a personal training program (S109). The device 1200 may store the personal training program in the memory 1230 or 1232 of the device 1200, or the device 1200 may transfer the personal training program to an external device.

The device 1200 may store the user's performance results for the stored personal training program and transmit them to an external device.

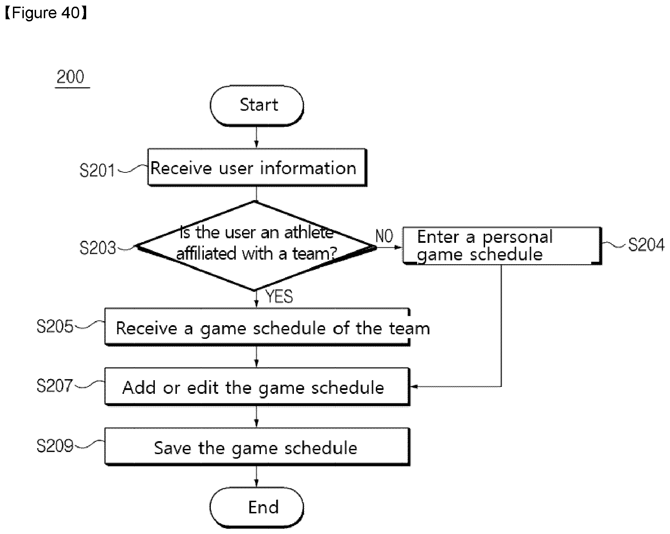

Next, referring to FIG. 40, a method of operating a device executing a performance enhancing application (PEA) of the present disclosure is described.

When the performance enhancement application (PEA) is launched, the device 1200 may receive input of information from a user attempting to utilize the application (S201). The input of user information may be an attempt by the user to log in to the application. When the user logs into an account through the application, the device 1200 may receive the user's information from the server.

The device 1200 receiving the user information may determine, based on the user information, whether the user is an athlete affiliated with a team(S203). If the user is affiliated with a team, the device 1200 may receive a game schedule for the team the user is on (S205). The device 1200 may request a game schedule of the user's team from a server, and receive a game schedule transmitted from the server in response to the request. The device 1200 may provide the game preparation program as a recommendation based on the received game schedule of the user.

If the user is an individual athlete not affiliated with a team, the device 1200 may receive input of the user's personal game schedule (S204). In one embodiment, the device 1200 may generate a recommended game schedule based on the game schedule entered by the user and present it to the user. For example, if the user adds a game A on November 23, 2023 as a schedule, the device 1200 may recommend to the user a game a1 on March 21, 2023, a game a2 on June 21, 2023, and so on that is associated with game A. As another example, the device 1200 may learn the user's training routine based on the game schedule entered by the user, and may provide the user with recommended game schedules.

The device 1200 may receive input to add or modify a game schedule (S207). The addition or modification of the game schedule may come directly from the athlete, or may be driven by a request received by the coach's device. Step 207 allows the game schedule to be customized for different athletes, and in some embodiments, step 207 may be omitted.

The device 1200 may store a game schedule (S209). The device 1200 may store the game schedule in memory 1230 or 1232 of the device 1200, or may transmit the game schedule to an external device.

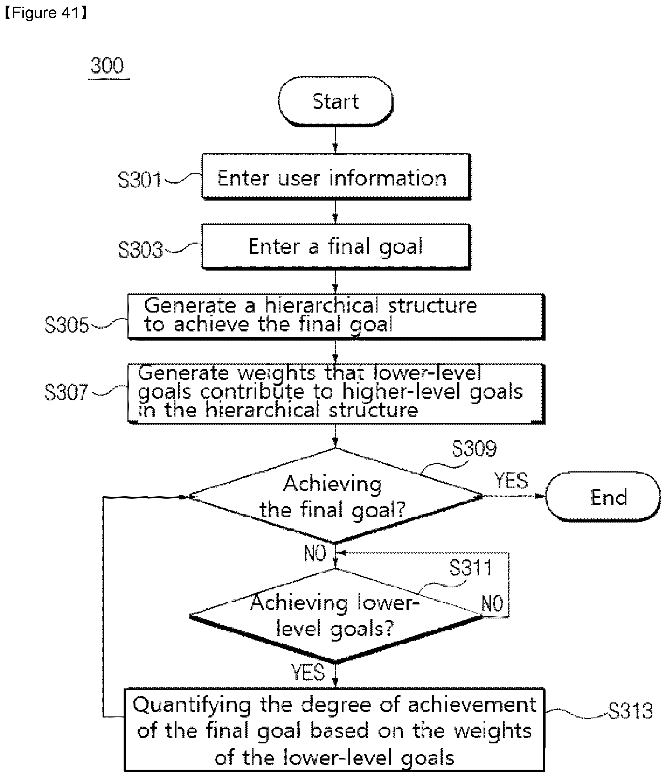

Finally, referring to FIG. 41, a method of operation of a device executing a performance enhancing application (PEA) of the present disclosure is described.

When the performance enhancement application (PEA) is launched, the device 1200 may receive input of information from a user attempting to utilize the application (S301). The input of the user information may be an attempt by the user to log in to the application. When the user logs into an account through the application, the device 1200 may receive the user's information from the server.

After receiving the user information, the device 1200 may receive an final goal input from the user (S303). Based on the inputted final goal, the device may generate a hierarchical structure for achieving the final goal (S305). For example, if the user enters attending college A as an final goal, the device 1200 may set attending college A as a level 5 final goal, and may set winning game a, which is required to attend college A, and winning game b, which is a sub-level of level 5, as level 4 goals. Further, the device 1200 may set breaking the first through third records for winning the a and b games as a Level 3 goal, which is a sub-level of Level 4. The device 1200 may set mastering a specific skill as a level 2 goal, a sub-level of level 3, for breaking the first to third records, and may set a training program, stretching, massage, etc. as a level 1 goal, a sub-level of level 2, for mastering a specific skill.

The device 1200 may generate weights (S307) that lower-level goals in the generated hierarchical structure contribute to higher-level goals. For example, the device 1200 may set the weights of winning game a and winning game b, which are required for the final goal of attending college A, to be 0.6 and 0.4, respectively. The device 1200 may generate weights for all lower-level goals in the hierarchical structure.

The device 1200 may determine if the user has achieved the final goal (S309) and, if not, may determine if the user has achieved a lower-level goal (S311). If the user has achieved the lower-level goal, the device 1200 may quantify the degree to which the user has achieved the final goal based on weights of the lower-level goals (S313). The device 1200 may receive input from the user that one of the lower-level goals has been achieved, and may quantify the degree of achievement of the final goal based on weights of the lower-level goals. For example, the device 1200 may quantify the achievement of the final goal of attending college A as 60% if the weight of winning a game required to attend college A is 0.6 and the user has won a game. The device 1200 may provide the quantified result to the user. The user can view the quantified achievement rate and visibly see how they are performing toward their final goal.

While features and elements have been described above in specific combinations, those skilled in the art will recognize that each feature or element may be used alone or in any combination with other features and elements. Furthermore, the methods described herein may be implemented as computer programs, software, or firmware incorporated into a computer-readable medium for execution by a computer or processor. Examples of computer-readable media include electronic signals (transmitted over a wired or wireless connection) and computer-readable storage media. Examples of computer-readable storage media include, but are not limited to, read-only memory (ROM), random access memory (RAM), registers, cache memory, semiconductor memory devices, magnetic media, such as internal hard disks and removable disks, optical-magnetic media, and optical media, such as CD-ROM disks and digital versatile disks (DVDs). The processor associated with the software may be used to implement a radio frequency transceiver for use in a WTRU, UE, terminal, base station, RNC, or any host computer.

In another aspect, the present disclosure relates to the following embodiments.

Embodiment 1. A method for use in a device for improving performance of a user, the method comprising,
generating one or more performance enhancing tasks, the one or more performance enhancing tasks causing at least one physiological change in a physiological mechanism of the user to cope with at least one of anxiety or stress, based on the user's performance in the one or more performance enhancing tasks; and
Includes the steps of providing one or more performance improvement challenges to a user via a device.

Embodiment 2. The method of embodiment 1, wherein the at least one physiological change comprises at least one of inducing dopamine secretion, inducing oxytocin secretion, reducing cortisol levels, inducing melatonin secretion, or facilitating synapses.

Example 3. The method of example 1 or example 2, wherein the one or more performance enhancing tasks include a first focus enhancing digital task, a second focus enhancing digital task, an anxiety/stress reducing digital task, and a coordination & agility enhancing digital task.

Example 4. The method of Example 3, wherein the first focus-enhancing digital challenge comprises positive thoughts, accomplishments, and completions designed to cause the induction of dopamine release in the user's physiological mechanisms.

Embodiment 5. The method of embodiment 3, wherein the second focus-enhancing digital challenge comprises a sleep cycle designed to cause an induction of melatonin secretion in a physiological mechanism of the user.

Embodiment 6. The method of embodiment 3, wherein the anxiety/stress reduction digital challenge comprises music designed to cause at least one of inducing oxytocin secretion or reducing cortisol levels in a physiological mechanism of the user.

Example 7. The method of Example 3, wherein the coordination & agility enhancing digital challenge includes a life cycle, regular exercise and rest, and no isolation designed to cause synaptic triggering in the user's physiological mechanisms.

Example 8. The method of example 1, wherein the user is an athlete or patient who has undergone surgery for an intraocular lens/multifocal intraocular lens (IOL) implant in an ophthalmology practice.

Embodiment 9. An apparatus for improving a user's performance, the apparatus comprising a processor, the processor generating one or more performance enhancing tasks - the one or more performance enhancing tasks causing at least one physiological change in a physiological mechanism of the user to cope with at least one of anxiety or stress, based on the user's performance in the one or more performance enhancing tasks -; and configured to provide the one or more performance enhancing tasks to the user.

Embodiment 10. The device of embodiment 9, wherein the at least one physiological change comprises at least one of inducing dopamine secretion, inducing oxytocin secretion, reducing cortisol levels, inducing melatonin secretion, or facilitating synapses.

Example 11. The device of example 10, wherein the one or more performance enhancing tasks include a first focus enhancing digital task, a second focus enhancing digital task, an anxiety/stress reducing digital task, and a coordination & agility enhancing digital task.

Example 12. The device of Example 11, wherein the first focus-enhancing digital task includes positive thoughts, accomplishments, and completions designed to cause induction of dopamine release in the physiological mechanism of the user, and the second focus-enhancing digital task includes a sleep cycle configured to cause induction of melatonin release in the physiological mechanism of the user.

Embodiment 13. The device of embodiment 11, wherein the anxiety/stress reduction digital challenge comprises music designed to cause at least one of inducing oxytocin secretion or reducing cortisol levels in a physiological mechanism of the user.

Example 14. In the device of Example 11, the coordination & agility enhancing digital challenge includes a life cycle designed to cause synaptic facilitation in the user's physiological mechanisms, regular exercise and rest, and a sense of non-isolation.

Example 15. The device of Example 11, wherein the user is an athlete or patient who has undergone surgery for an intraocular lens/multifocal lens (IOL) implant in an ophthalmology practice.

Embodiment 16. A method for improving a performance of an athlete, the method comprising,

The method includes performing one or more performance enhancing tasks by an athlete that cause at least one physiological change in a physiological mechanism of the athlete to cope with at least one of anxiety or stress.

Embodiment 17. The method of embodiment 16, wherein the at least one physiological change comprises at least one of inducing dopamine secretion, inducing oxytocin secretion, reducing cortisol levels, inducing melatonin secretion, or facilitating synapses.

Example 18. The method of example 16, wherein the one or more performance enhancing tasks include a first focus enhancing digital task, a second focus enhancing digital task, an anxiety/stress reducing digital task, and a coordination & agility enhancing digital task.

Embodiment 19. A method for improving a patient's performance, the method comprising,

A method comprising: performing, by a patient, one or more performance enhancing tasks that cause at least one physiological change in a physiological mechanism of an athlete to cope with at least one of anxiety or stress, the at least one physiological change comprising at least one of: inducing dopamine secretion, inducing oxytocin secretion, reducing cortisol levels, inducing melatonin secretion, or facilitating synapses.

Example 20. The method of example 19, wherein the one or more performance enhancing tasks include a first focus enhancing digital task, a second focus enhancing digital task, an anxiety/stress reducing digital task, and a coordination & agility enhancing digital task.

Embodiment 21. A method for improving performance of a user, the method comprising,
providing to the user, by an electronic device, one or more first modules selected from the group consisting of a focus module I for inducing dopamine release, an anxiety and/or stress module, a focus module II for inducing melatonin release, and a coordination and agility module, each of the one or more first modules comprising one or more first tasks for the user to follow,
an electronic device comprising: (i) a sensor for detecting a user's compliance with a first task of the one or more first modules; (ii) transmitting compliance information based on the compliance information to a server; and (iii) receiving from the server one or more second tasks based on the compliance information; and

Providing to a user, by an electronic device, one or more second modules selected from the group consisting of a focus module I for inducing dopamine release, an anxiety and/or stress module, a focus module II for inducing melatonin release, and a coordination and agility module, the one or more second modules comprising one or more second tasks.

Embodiment 22. The method of embodiment 21, wherein the one or more first modules,
Includes Focus Module I to enhance the user's ability to focus, which includes one or more primary tasks related to positive thinking, achievement, and completion.

Embodiment 23. The method of embodiment 22, wherein the one or more tasks include one or more tasks for positive emotions, behavior maintenance, exercise, and training in a bright area.

Embodiment 24. The method of any one of embodiments 21 to 23, wherein the one or more first modules comprise an anxiety and/or stress module for alleviating anxiety and/or stress, the anxiety and/or stress module comprising one or more first tasks for inducing oxytocin secretion and/or lower cortisol levels.

Embodiment 25. The method of embodiment 24, wherein the one or more first tasks comprise one or more tasks to listen to music.

Embodiment 26. The method of embodiment 25, wherein the electronic device receives and plays a sound for music.

Embodiment 27. The method of any one of embodiments 21 to 26, wherein the one or more first modules comprise a concentration module II for enhancing the user's concentration, the concentration module II comprising one or more first tasks related to the sleep cycle.

Embodiment 28. The method of embodiment 27, wherein the one or more tasks include one or more tasks for balanced life cycle and brightness of the user's environment.

Embodiment 29. The method of any one of embodiments 21 to 28, wherein the one or more first modules comprise a coordination and agility module for improving coordination and agility, the coordination and agility module comprising one or more first tasks for promoting synchronization.

Embodiment 30. The method of embodiment 29, wherein the one or more first challenges include one or more challenges for a balanced life cycle, regular exercise and rest, brightness of the user's environment, and non-isolation or numbness.

Embodiment 31. The method of any one of embodiments 21 to 30, wherein the server receives one or more second assignments from an external reviewer.

Embodiment 32. The method of embodiment 31, wherein the external reviewer includes a health professional.

Embodiment 33. The method of embodiment 31, wherein the external reviewer comprises artificial intelligence (Al).

Embodiment 34. The method of any one of embodiments 21 to 33,

The sensor includes one or more of a motion sensor, a camera, an accelerometer, a magnetometer, a light sensor, a microphone, a proximity sensor, a touch sensor, a gyroscope, a global positioning system (GPS) sensor, an ambient light sensor, a fingerprint sensor, a pedometer, a heart rate sensor, and a thermometer.

Embodiment 35. The method of any one of embodiments 21 to 34, wherein the sensor comprises a GPS sensor and a heart rate sensor.

Embodiment 36. The method of any one of embodiments 21 to 35, wherein the sensor comprises a touch sensor, and the user uses the touch sensor to provide compliance information to the electronic device.

Embodiment 37. The method of any one of embodiments 21 to 36, wherein the user is a musician and the performance is a musical performance.

Example 38. The method of any one of examples 21 to 36, wherein the user is an athlete and the performance is an athletic performance.

Example 39. The method of any one of Examples 21 to 36, wherein the user is a baseball player.

Embodiment 40. The method of any one of embodiments 21 to 36, wherein the user is a skier.

Embodiment 41. The method of any one of embodiments 38 to 40, wherein the sensor captures motion of an athletic performance.

Embodiment 42. The method of embodiment 41, wherein the sensor captures at least 30 images per second.

Embodiment 43. A system for improving performance of a user, the system comprising,
a digital device configured to execute a digital application comprising one or more first modules for improving a user's performance, the digital device including sensors for detecting the user's compliance with a first set of tasks of the one or more first modules;
a healthcare provider portal configured to provide the healthcare provider with one or more options for performing one or more tasks that prescribe improvements to the user's performance based on information received from the digital application; and
Includes an administrative portal configured to provide the system's administrator with one or more options for performing one or more tasks to manage access to the system by healthcare providers.

Example 44. The system of example 43, wherein the digital application is
Instruct the processor of the digital device to execute actions, including actions to create a digital therapeutic module to improve performance based on the therapeutic hypothesis and mechanism of action (MOA) to improve the user's performance.

Example 45. The system of example 44, wherein generating the digital therapeutic module comprises generating the digital therapeutic module based on a biochemical factor associated with improving the user's performance.

Embodiment 46. The system of any one of embodiments 43 to 45, wherein the one or more first modules are selected from the group consisting of a focus module I for inducing dopamine release, an anxiety and/or stress module, a focus module II for inducing melatonin release, and a coordination and agility module.

Embodiment 47. The system of any one of embodiments 43 to 46, wherein the one or more first modules include a focus module I for enhancing the user's focus, and wherein the focus module I includes one or more first tasks related to positive thinking, accomplishment, and completion.

Example 48. The system of Example 47, wherein the one or more tasks include one or more tasks for positive emotions, behavior maintenance, exercise, and training in a bright area.

Embodiment 49. The system of any one of embodiments 46 to 48, wherein the one or more first modules comprise an anxiety and/or stress module for alleviating anxiety and/or stress, the anxiety and/or stress module comprising one or more first tasks for inducing oxytocin secretion and/or lower cortisol levels.

Example 50. The system of example 49, wherein the one or more first tasks include one or more tasks to listen to music.

Example 51. In the system of example 50, the electronic device receives and plays a sound for music.

Embodiment 52. The system of any one of embodiments 46 to 51, wherein the one or more first modules include a concentration module II for enhancing the user's concentration, and the concentration module II includes one or more first tasks related to the sleep cycle.

Embodiment 53. The system of embodiment 52, wherein the one or more challenges include one or more challenges for a balanced life cycle and brightness of the user's environment.

Embodiment 54. The system of any one of embodiments 46 to 53, wherein the one or more first modules comprise a coordination and agility module for improving coordination and agility, the coordination and agility module comprising one or more first tasks for facilitating synchronization.

Embodiment 55. The system of embodiment 54, wherein the one or more first challenges include one or more challenges for a balanced life cycle, regular exercise and rest, brightness of the user's environment, and non-isolation or numbness.

Embodiment 56. In the system of any one of embodiments 43 to 55, the server receives one or more second assignments from an external reviewer.

Embodiment 57. The system of any one of embodiments 43 to 56, wherein the external reviewer includes a health professional.

Embodiment 58. The system of any one of embodiments 43 to 57, wherein the external reviewer comprises artificial intelligence (Al).

Embodiment 59. The system of any one of embodiments 43 to 58,

The sensor includes one or more of a motion sensor, a camera, an accelerometer, a magnetometer, a light sensor, a microphone, a proximity sensor, a touch sensor, a gyroscope, a global positioning system (GPS) sensor, an ambient light sensor, a fingerprint sensor, a pedometer, a heart rate sensor, and a thermometer.

Example 60. The system of any one of Examples 43 through 59, wherein the sensors include a GPS sensor and a heart rate sensor.

Embodiment 61. The system of any one of embodiments 43 to 60, wherein the sensor comprises a touch sensor, and the user uses the touch sensor to provide compliance information to the electronic device.

Embodiment 62. The system of any one of embodiments 43 to 61, wherein the user is a musician and the performance is a musical performance.

Example 63. The system of any one of Examples 43 to 61, wherein the user is an athlete and the performance is an athletic performance.

Example 64. The system of any one of Examples 43 to 61, wherein the user is a baseball player.

Embodiment 65. The system of any one of embodiments 43 to 61, wherein the user is a skier.

Embodiment 66. The system of any one of embodiments 63 to 65, wherein the sensor captures motion of an athletic performance.

Example 67. The system of Example 66, wherein the sensor captures at least 30 images per second.

Embodiment 68. The system of any one of embodiments 43 to 67, wherein the one or more options provided to the healthcare provider are selected from the group consisting of: adding or removing a user; viewing or editing personal information about the user; viewing adherence information about the user; viewing the user's results for one or more at least partially completed digital therapeutic modules; prescribing one or more digital therapeutic modules to the user; changing a prescription for one or more digital therapeutic modules; and communicating with the user.

Embodiment 69. The system of embodiment 68, wherein the one or more options comprise viewing or editing personal information about the user, the personal information comprising one or more pieces of information selected from the group consisting of an identification number for the user, a name of the user, a date of birth of the user, an email of the user, an email of a guardian of the user, a contact phone number for the user, a prescription for the user, and one or more notes written about the user by a healthcare provider.

Embodiment 70. The system of embodiment 69, wherein the personal information includes a prescription for the user, the prescription for the user comprising at least one selected from the group consisting of a prescription identification number, a prescription type, a start date, a duration, a completion date, a number of scheduled or prescribed digital therapy modules to be performed by the user, and a number of scheduled or prescribed digital therapy modules to be performed by the user in a day.

Embodiment 71. The system of any one of embodiments 43 to 70, wherein the one or more options include viewing adherence information, the user's adherence information comprising one or more of a plurality of scheduled or prescribed digital therapy modules completed by the user, and a calendar identifying one or more dates on which the user completed, partially completed, or did not complete the one or more scheduled or prescribed digital therapy modules.

Embodiment 72. The system of any one of embodiments 43 to 71 , wherein the one or more options include viewing a user's results, the user's results for the one or more at least partially completed digital therapy modules comprising one or more selected from the group consisting of a time the user started the scheduled or prescribed digital therapy module, a time the user stopped the scheduled or prescribed digital therapy module, an indicator of whether the scheduled or prescribed digital therapy module was fully or partially completed, and an exercise intensity (EI).

Example 73. The system of any one of Examples 43 to 72, wherein the one or more options provided to the administrator of the system are selected from the group consisting of adding or removing a healthcare provider, viewing or editing personal information about the healthcare provider, viewing or editing de-identified information about the user, viewing adherence information about the user, viewing the user's results for one or more at least partially completed digital therapeutic modules, and communicating with the healthcare provider.

Embodiment 74. The system of embodiment 73, wherein the one or more options include viewing or editing personal information, and wherein the personal information of the healthcare provider includes one or more selected from the group consisting of an identification number for the healthcare provider, a name of the healthcare provider, an email of the healthcare provider, and a contact phone number of the healthcare provider.

Embodiment 75. The system of embodiment 73 or embodiment 74, wherein the one or more options include viewing or editing de-identified information of the user, the de-identified information of the user including one or more selected from the group consisting of an identification number for the user and a health care provider for the user.

Embodiment 76. The system of any one of embodiments 43 to 75, wherein the one or more options include viewing adherence information for the user, the adherence information for the user comprising one or more of a plurality of scheduled or prescribed digital therapy modules completed by the user, and a calendar identifying one or more dates on which the user completed, partially completed, or did not complete the one or more scheduled or prescribed digital therapy modules.

Embodiment 77. The system of any one of embodiments 43 to 76, wherein the one or more options include viewing a user's results, the user's results for the one or more at least partially completed digital therapy modules comprising one or more selected from the group consisting of a time the user started the scheduled or prescribed digital therapy module, a time the user stopped the scheduled or prescribed digital therapy module, an indicator of whether the scheduled or prescribed digital therapy module was fully or partially completed, and an exercise intensity (EI).

Example 78. The system of any one of Examples 43 to 77, wherein the digital application further comprises push alarms and/or push notifications to remind the subject to complete the digital therapeutic module.

Embodiment 79. The system of any one of embodiments 43 to 78, wherein the digital device is,
a digital assignment generator configured to create a digital therapeutic module to improve a user's performance, create a digital assignment based on the digital therapeutic module, and deliver the digital assignment to the user; and
Contains a results collection configured to collect the results of a user's execution of a digital challenge.

Example 80. The system of Example 79, wherein the digital assignment generation part generates a digital therapeutic module based on input from the healthcare provider.

Example 81. In the system of example 79 or example 80, the digital assignment generation part generates a digital therapy module based on information received from the user.

Embodiment 82. The system of embodiment 81, wherein the information received from the user comprises at least one of the user's underlying factors, medical information, and digital therapeutic literacy,

Baseline factors include your activity, heart rate, sleep, and diet,

Medical information includes your electronic medical record (EMR), family history, genetic vulnerabilities, and genetic susceptibilities; and
Digital therapeutics literacy includes user accessibility and technology adoption of digital therapeutics and devices.

Embodiment 83. The system of any one of embodiments 79 to 82, wherein the result collection part collects the results of the execution of the digital task by monitoring the user's compliance with the digital task or by allowing the user to directly enter the user's compliance with the digital task.

Embodiment 84. The system of any one of embodiments 79 to 83, wherein generating the digital assignment at the digital assignment generation part and collecting the results of user execution of the digital assignment at the result collection part are executed multiple times in multiple feedback loops; and

The Create Digital Assignment part creates a user's digital assignment for the current cycle based on the user's digital assignment from the previous cycle and the execution result data for the user's digital assignment from the previous cycle collected in the Results Collection part.

Embodiment 85. A computing system for improving performance of a user, the computing system,
a display configured to present to a user one or more first modules selected from the group consisting of a focus module I for inducing dopamine release, an anxiety and/or stress module, a focus module II for inducing melatonin release, and a coordination and agility module, each of the one or more first modules comprising one or more first tasks for the user to follow;
A sensor configured to detect a user's compliance with a task in one or more of the first modules;
A transmitter configured to send compliance information to the server based on the level of compliance; and
Includes a listener configured to receive one or more second assignments from the server based on compliance information,

The display is further configured to provide the user with one or more second modules selected from the group consisting of a focus module I for inducing dopamine release, an anxiety and/or stress module, a focus module II for inducing melatonin release, and a coordination and agility module, each of the one or more second modules comprising one or more second tasks.

Embodiment 86. The computing system of embodiment 85, wherein the digital application for improving performance,
Instructs the processor of a digital device to execute an action, including an action to create a digital therapeutic module to improve performance based on a therapeutic hypothesis and a mechanism of action (MOA) to improve performance.

Embodiment 87. The computing system of embodiment 86, wherein generating the digital therapeutic module comprises generating the digital therapeutic module based on a biochemical factor associated with improved performance.

Embodiment 88. The computing system of any one of embodiments 85 to 87, wherein the one or more first modules are selected from the group consisting of a focus module I for inducing dopamine release, an anxiety and/or stress module, a focus module II for inducing melatonin release, and a coordination and agility module.

Embodiment 89. The computing system of any one of embodiments 85 to 88, wherein the one or more first modules,
Includes Focus Module I to enhance the user's ability to focus, which includes one or more primary tasks related to positive thinking, achievement, and completion.

Embodiment 90. The computing system of embodiment 89, wherein the one or more tasks include one or more tasks for positive emotions, behavior maintenance, exercise, and training in a bright area.

Embodiment 91. The computing system of any one of embodiments 85 to 90, wherein the one or more first modules comprise an anxiety and/or stress module for alleviating anxiety and/or stress, the anxiety and/or stress module comprising one or more first tasks for inducing oxytocin secretion and/or lower cortisol levels.

Embodiment 92. The computing system of embodiment 91, wherein the one or more first tasks include one or more tasks to listen to music.

Example 93. In the computing system of example 92, the electronic device receives and plays a sound for music.

Embodiment 94. The computing system of any one of embodiments 85 to 93, wherein the one or more first modules comprise a concentration module II for enhancing a user's concentration, the concentration module II comprising one or more first tasks related to a sleep cycle.

Embodiment 95. The computing system of embodiment 94, wherein the one or more challenges include one or more challenges for a balanced life cycle and brightness of the user's environment.

Embodiment 96. The computing system of any one of embodiments 85 to 95, wherein the one or more first modules comprise a coordination and agility module for improving coordination and agility, the coordination and agility module comprising one or more first tasks for facilitating synchronization.

Embodiment 97. The computing system of embodiment 96, wherein the one or more first challenges include one or more challenges for a balanced life cycle, regular exercise and rest, brightness of the user's environment, and non-isolation or numbness.

Embodiment 98. In the computing system of any one of embodiments 85 through 97, the server receives the one or more second assignments from the external reviewer.

Embodiment 99. In the computing system of embodiment 98, the external reviewer includes a health professional.

Embodiment 100. In the computing system of embodiment 98, the external reviewer includes artificial intelligence (Al).

Embodiment 101. The computing system of any one of embodiments 85 to 100, wherein the sensors include one or more of: a motion sensor, a camera, an accelerometer, a magnetometer, a light sensor, a microphone, a proximity sensor, a touch sensor, a gyroscope, a global positioning system (GPS) sensor, an ambient light sensor, a fingerprint sensor, a pedometer, a heart rate sensor, and a thermometer.

Embodiment 102. The computing system of any one of embodiments 85 to 101, wherein the sensors include a GPS sensor and a heart rate sensor.

Embodiment 103. In the computing system of any one of embodiments 85 to 102, the sensor comprises a touch sensor, and the user uses the touch sensor to provide compliance information to the electronic device.

Embodiment 104. The computing system of any one of embodiments 85 to 103, wherein the user is a musician and the performance is a musical performance.

Embodiment 105. The computing system of any one of embodiments 85 to 103, wherein the user is an athlete and the performance is an athletic performance.

Embodiment 106. The computing system of any one of embodiments 85 to 103, wherein the user is a baseball player.

Embodiment 107. In the computing system of any one of embodiments 85 to 105, the user is a skier.

Embodiment 108. In the computing system of any one of embodiments 105 to 107, the sensor captures motion of an athletic performance.

Embodiment 109. In the computing system of embodiment 108, the sensor captures at least 30 images per second.

Embodiment 110. The computing system of any one of embodiments 85 to 109, wherein the digital application further comprises push alarms and/or push notifications to remind the subject to complete the digital therapeutic module.

Embodiment 111. The computing system of any one of embodiments 85 to 110, wherein the digital device is,
a digital assignment generator configured to create a digital therapeutic module to improve a user's performance, create a digital assignment based on the digital therapeutic module, and deliver the digital assignment to the user; and
Contains a results collection configured to collect the results of a user's execution of a digital challenge.

Example 112. In the computing system of example 111, the digital assignment generation part generates a digital therapeutic module based on input from the healthcare provider.

Example 113. In the computing system of example 111 or example 112, the digital assignment generation part generates a digital therapy module based on information received from the user.

Embodiment 114. The computing system of embodiment 113, wherein the information received from the user includes at least one of the user's underlying factors, medical information, and digital therapeutic literacy,
Baseline factors include your activity, heart rate, sleep, and diet,
Medical information includes your electronic medical record (EMR), family history, genetic vulnerabilities, and genetic susceptibilities,
Digital therapeutics literacy includes user accessibility and technology adoption of digital therapeutics and devices.

Embodiment 115. In the computing system of any one of embodiments 111 to 114, the result collection portion collects the results of the execution of the digital task by monitoring the user's compliance with the digital task or by allowing the user to directly enter the user's compliance with the digital task.

Embodiment 116. The computing system of any one of embodiments 111 to 115, wherein generating the digital task at the digital task generation part and collecting the results of user execution of the digital task at the result collection part are executed multiple times in multiple feedback loops; and

The Create Digital Assignment part creates a user's digital assignment for the current cycle based on the user's digital assignment from the previous cycle and the execution result data for the user's digital assignment from the previous cycle collected in the Results Collection part.

Embodiment 117. a non-transitory computer-readable medium storing software instructions for improving a user's performance as described above, the software instructions, when executed by a processor, causing the processor to,
causing the one or more first modules described above to be displayed to a user by an electronic device, each of the one or more first modules comprising a task for the user to follow;
wherein the electronic device detects the user's compliance with the tasks of the one or more first modules by sensors as described above;
Enable the electronic device to send compliance information based on the level of compliance to a server;
Receive one or more secondary assignments from the server based on the compliance information; and
Causes one or more second modules described above to be displayed to the user, wherein the one or more second modules include one or more second assignments.

Embodiment 118. The method of any of embodiments 21 to 42, the system of any of embodiments 43 to 84, the computing system of any of embodiments 85 to 116, or the non-transitory computer-readable medium of embodiment 117, wherein the one or more first modules comprise a focus module I for inducing dopamine release, an anxiety and/or stress module, a focus module II for inducing melatonin release, and a coordination and agility module.

Embodiment 119. The method of any one of embodiments 21 to 42, the system of any one of embodiments 43 to 84, the computing system of any one of embodiments 85 to 116, or the non-transitory computer-readable medium of embodiment 117, wherein the adherence is adherence to the first task of the concentration module I, and wherein the adherence information based on the adherence to the first task of the concentration module I is used to generate only the second task of the concentration module I.

Embodiment 120. The method of any of embodiments 21 to 42, the system of any of embodiments 43 to 84, the computing system of any of embodiments 85 to 116, or the non-transitory computer-readable medium of embodiment 117, wherein the adherence is adherence to the first task of the anxiety and/or stress module, and wherein the adherence information based on the adherence to the first task of the anxiety and/or stress module is used to generate only the second task of the anxiety and/or stress module.

Embodiment 121. The method of any one of embodiments 21 to 42, the system of any one of embodiments 43 to 84, the computing system of any one of embodiments 85 to 116, or the non-transitory computer-readable medium of embodiment 117, wherein the adherence is adherence to the first task of the intensive module II, and wherein the adherence information based on the adherence to the first task of the intensive module II is used to generate only the second task of the intensive module II.

Embodiment 122. The method of any of embodiments 21 to 42, the system of any of embodiments 43 to 84, the computing system of any of embodiments 85 to 116, or the non-transitory computer-readable medium of embodiment 117, wherein the compliance is compliance with the first task of the coordination and agility module, and wherein the compliance information based on the compliance with the first task of the coordination and agility module is used to generate only the second task of the coordination and agility module.

## Claims

1. A digital apparatus for enhancing performance of a user, comprising:
at least one processor; and
at least one memory that stores an application to enhance performance of a user,
wherein the at least one processor by executing the application, receives user information from a first user;
generates a recommended program to enhance performance of the first user based on the user information;
provides the recommended program to the first user; and
stores a training program of the first user based on the first user's response.

2. The digital apparatus of claim 1,
wherein the user information comprises the at least one of the first user's goals, or the first user's game schedule;
wherein the first user's training program comprises training for physical health of the first user and training for mental health of the first user.

3. The digital apparatus of claim 2,
wherein the training for the mental health of the first user comprises at least one of meditation, muscle stretching, motivation, image training, and listening to music.

4. The digital apparatus of claim 3, wherein the at least one processor,
receives content information for the image training from the first user; and
provides recommended content by analyzing the content information

5. The digital apparatus of claim 1, wherein the at least one processor,
determines whether the first user is a player belongs to a team based on the user information;
receives a training program of the team to which the first user belongs if the first user is a player belonging to the team; and
generates the recommended program based on the training program of the team.

6. The digital apparatus of claim 1, wherein the at least one processor,
receives input from the first user to add or modify the training program of the first user; and
transmits a request for permission to add or modify the training program of the first user to a second user's digital device.

7. The digital apparatus of claim 1, wherein the at least one processor,
receives a request from the second user's digital device to add or modify the training program of the first user;
receives a response from the first user to the request to add or modify the training program of the first user; and
adds or modifies the training program of the first user based on the response.

8. The digital apparatus of claim 1, wherein the at least one processor,
stores results of performing the training program by the first user; and
transmits the performing results to the second user's digital device.

9. The digital apparatus of claims 6 to 8,
wherein the first user is an athlete, and the second user is a coach of the first user.

10. A digital apparatus for enhancing performance of a user, comprising:
at least one processor; and
at least one memory that stores an application to enhance performance of a user,
wherein the at least one processor by executing the application,
receives user information from a first user;
receives a final goal of the first user;
generates a hierarchical structure to achieve the final goal;
generates weights that lower-level goals contribute to higher-level goals in the hierarchical structure; and
provides the first user with a training program for achieving the final goal.

11. The digital apparatus of claim 10, wherein the at least one processor,
receives input from the first user indicating that one of the lower-level goals has been achieved; and
provides quantification of the degree of achievement of the final goal based on the weights of the lower-level goals.

12. A method for use in an apparatus to enhance performance of a user, the method comprising:
receiving user information from a first user;
generating a recommended program to enhance the performance of the first user based on the user information;
providing the recommended program to the first user; and
storing a training program of the first user based on the first user's response.

13. A computer-readable medium having stored a program for executing the method of claim 12 on a computer.

14. The method for use in an apparatus to enhance performance of a user, the method comprising:
receiving user information from the first user;
receiving a final goal of the first user;
generating a hierarchical structure to achieve the final goal;
generating weights that lower-level goals contribute to higher-level goals in the hierarchical structure; and
providing a training program to the first user for achieving the final goal.

15. A computer-readable medium having stored a program for executing the method of claim 14 on a computer.
